# EUROPÄISCHE PATENTSCHRIFT

(11) **EP 3 573 698 B1**
(45) Veröffentlichungstag und Bekanntmachung des Hinweises auf die Patenterteilung: **21.06.2023**
(21) Anmeldenummer: 18702263.7
(22) Anmeldetag: 29.01.2018
(51) Int. Cl.: A61M 39/18, A61M 39/10

(54) **VORRICHTUNG UND VERFAHREN ZUM HERSTELLEN UND/ODER LÖSEN EINER MIT EINEM FLUID DURCHSTRÖMBAREN, MEDIZINISCHEN GEWINDEVERBINDUNG**
APPARATUS AND METHOD FOR ESTABLISHING AND/OR RELEASING A MEDICAL THREADED CONNECTION THROUGH WHICH A FLUID CAN FLOW
DISPOSITIF ET PROCÉDÉ POUR LA CRÉATION ET/OU LE DESSERRAGE D'UN RACCORD FILETÉ MÉDICAL À TRAVERS LEQUEL PEUT S'ÉCOULER UN FLUIDE

(30) Priorität: 30.01.2017 DE 102017201447
(43) Veröffentlichungstag der Anmeldung: 04.12.2019
(73) Patentinhaber: Fresenius Medical Care Deutschland GmbH, 61352 Bad Homburg (DE)
(72) Erfinder: ARKOSSY, Otto, 1037 Budapest (HU); DANIEL, Pia, 78351 Bodman (DE); HAUKE, Christopher, 55246 Mainz-Kostheim (DE); KOPPERSCHMIDT, Pascal, 97456 Dittelbrunn (DE); RITTER, Kai-Uwe, 91126 Rednitz-Hembach (DE); SCHOLZ, Cäcilia, 65824 Schwalbach (DE); SCHULTE, Elke, 97422 Schweinfurt (DE); SPICKERMANN, Reiner, 97535 Wasserlosen-Burghausen (DE)
(74) Vertreter: Ricker, Mathias
(86) Internationale Anmeldenummer: PCT/EP2018/052117
(87) Internationale Veröffentlichungsnummer: WO 2018/138327

(56) Entgegenhaltungen:
- WO-A2-2004/047883
- WO-A2-2005/000378
- US-A1- 2008 287 859

## Beschreibung

Die Erfindung betrifft eine Vorrichtung zum Herstellen und/oder Lösen einer mit einem Fluid durchströmbaren, medizinischen Gewindeverbindung aus einem ersten Verbindungselement und einem zweiten Verbindungselement, insbesondere zum Herstellen und/oder Lösen einer Luer-Lock-Verbindung, wobei die Vorrichtung eine erste Aufnahmeeinrichtung zur Aufnahme des ersten Verbindungselements und eine zweite Aufnahmeeinrichtung zur Aufnahme des zweiten Verbindungselements aufweist, wobei die erste Aufnahmeeinrichtung und die zweite Aufnahmeeinrichtung um eine gemeinsame Verschraubungsachse relativ zueinander drehbar sind und entlang der Verschraubungsachse relativ zueinander verlagerbar sind, und wobei die Vorrichtung dazu eingerichtet ist, nach dem Einbringen des ersten Verbindungselementes in die erste Aufnahmeeinrichtung und dem Einbringen des zweiten Verbindungselementes in die zweite Aufnahmeeinrichtung, die erste Aufnahmeeinrichtung und die zweite Aufnahmeeinrichtung derart relativ zueinander zu bewegen, dass das erste Verbindungselement und das zweite Verbindungselement miteinander verschraubt werden oder die Gewindeverbindung gelöst wird.

Zur medizinischen Behandlung vieler Patienten ist es erforderlich, zur Verabreichung von Medikamenten und/oder zur Herstellung einer fluiden Verbindung zwischen dem Blutkreislauf des Patienten und einem externen Fluidsystem einen Gefäßzugang zu legen. Insbesondere zur Punktion von Blutgefäßen werden sogenannte "Kanülen" eingesetzt.

Kanülen sind in der Regel Hohlnadeln, welche üblicherweise an ihrem einen Ende eine für den vorgesehen Einsatzzweck entsprechend ausgebildete Spitze aufweisen, insbesondere eine für den jeweiligen Einsatzzweck speziell geschliffene Spitze, oft eine sehr scharfe und spitze Spitze, bei Injektionsnadeln meistens eine Spitze mit Facettenschliff oder Lancettenschliff, und die an ihrem anderen Ende üblicherweise jeweils ein Verbindungselement zum Herstellen einer Fluidverbindung mit dem übrigen Teil des externen Fluidsystems, einer Spritze, einem Dosierbehälter, einem medizinischen Gerät oder dergleichen aufweisen. Bei den meisten Kanülen ist das Verbindungselement dabei ein sogenanntes Luer-Verbindungselement oder ein sogenanntes Luer-Lock-Verbindungselement, welches mit einem korrespondierend ausgebildeten, passenden Gegenstück verbindbar ist, wobei das Gegenstück zu einem Luer-Verbindungselement ein korrespondierend ausgebildetes Luer-Verbindungselement ist und das passende Gegenstück zu einem Luer-Lock-Verbindungselement ein korrespondierend ausgebildetes Luer-Lock-Verbindungselement.

Luer-Gewindeverbindungen, die in der Regel ein männliches Luer-Verbindungselement sowie ein weibliches Luer-Verbindungselement aufweisen, sind aus dem Stand der Technik grundsätzlich bekannt. Insbesondere sind verschiedenste Ausführungsformen von Luer-Verbindungselementen mit einem männlichen und/oder einem weiblichen Luer-Verbindungsabschnitt bekannt, beispielsweise aus der DE 10 2015 211 370 B3, welche sich insbesondere hinsichtlich ihrer Außenkontur sowie weiterer, vorhandener Anschlüsse unterscheiden. Patentdokumente US 2008/287859 A1 und WO 2005/000378 A2 offenbaren teilweise die Merkmale, die in Anspruch 1 beansprucht sind.

Luer-Gewindeverbindungen sind international in der Norm ISO 594 bzw. europaweit in der Norm EN 205953 (bzw. EN 1707) und deutschlandweit in der DIN EN 20594 normiert. Die Verwendung von Luer-Gewindeverbindungen ermöglicht die Herstellung von herstellerunabhängigen, sicheren, dichten und sterilen Verbindungen, wie sie insbesondere im medizintechnischen Bereich erforderlich sind. Die genormten Standard-Luer-Verbindungselemente unterschiedlicher Hersteller sind dabei beliebig miteinander kombinierbar. Somit können zur Herstellung einer Luer-Gewindeverbindung Spritzen und Kanülen unterschiedlicher Hersteller miteinander kombiniert werden.

Ein männliches Luer-Verbindungselement weist dabei einen ein Lumen umschließenden Rohrkörper mit einem konischen Außenmantel auf, wobei der konische Außenmantel gemäß der Norm von der Spitze zur Basis eine 6 %-Steigung aufweist.

Ein weibliches Luer-Verbindungselement weist einen korrespondierend zum konischen Außenmantel des männlichen Luer-Verbindungselementes ausgebildeten, hohlen Aufnahmekörper auf, der eine konische, hülsenförmige Aufnahme aufweist, welche ebenfalls eine 6 %-Steigung aufweist. Am äußeren Ende des weiblichen Luer-Verbindungselementes ist ferner ein Bund vorgesehen, der oftmals radial nach außen absteht. Das weibliche Luer-Verbindungselement lässt sich bei passend gewählter Größe leicht auf das männliche Luer-Verbindungselement aufstecken, wobei das weibliche Luer-Verbindungselement per Reibschluss sicher auf dem männlichen Luer-Verbindungselement gehalten werden kann und mit dem männlichen Luer-Verbindungselement eine fluiddichte Verbindung bereitstellt. Ebenso leicht wie das weibliche Luer-Verbindungselement mit dem männlichen Luer-Verbindungselement verbunden werden kann, kann die Verbindung jedoch wieder gelöst werden.

Aus diesem Grund sind neben den gewöhnlichen Luer-Verbindungen auch sogenannte Luer-Lock-Gewindeverbindungen entwickelt worden, welche ebenfalls grundsätzlich aus dem Stand der Technik bekannt sind. Diese weisen am männlichen Luer-Verbindungselement zusätzlich ein Innengewinde auf, in das radial vorstehende Verriegelungselemente, insbesondere ein Außengewinde eines korrespondierend ausgebildeten weiblichen Luer-Lock-Verbindungselementes, eingebracht werden können, mit Hilfe derer das weibliche Luer-Lock-Verbindungselement mit dem männlichen Luer-Lock-Verbindungselement verschraubt werden kann, so dass das männliche Luer-Lock-Verbindungselement und das weibliche Luer-Lock-Verbindungselement zumindest innerhalb gewisser Grenzen zugfest miteinander verbunden sind.

Üblicherweise befinden sich weibliche Luer- oder weibliche Luer-Lock-Verbindungselemente an einer Kanüle oder einem Katheter oder am Eingang einer Leitung. Männliche Luer- oder Luer-Lock-Verbindungselemente befinden sich meistens an einer Spritze, einem Dosierbehälter, einem medizinischen Gerät oder am Ausgang einer Leitung.

In der Regel sind sämtliche Fluidverbindungen zwischen einem Patienten und einem externen Fluidsystem, einer Spritze, einem Dosierbehälter, einem medizinischen Gerät oder dergleichen sowie oftmals eine Vielzahl der Fluidverbindungen innerhalb des externen, fluidisch mit dem Patienten verbundenen Fluidsystems Luer-Verbindungen oder Luer-Lock-Verbindungen.

Insbesondere bei Patienten mit einer chronischen Erkrankung, die zu ihrer Behandlung regelmäßig wiederholte Punktionen der Blutgefäße benötigen, ist es wichtig, dass die mechanischen Belastungen der Punktion eines Blutgefäßes, welche auch als "Kanülierung" bezeichnet wird, möglichst geringgehalten werden und die zu punktierenden Gefäße bei der Kanülierung so gut wie möglich geschont werden.

Dabei stellt nicht nur das Kanülieren an sich eine Belastung für das betreffende Gefäß dar, sondern jegliche mechanische Belastung des Gefäßes, insbesondere vor allem ein Bewegen der bereits in das Gefäß eingeführten Kanüle. Dies bedeutet, dass vor allem während des Verbindungsvorgangs, d.h. beim Verbinden der Kanüle mit dem übrigen Teil des externen Fluidsystems, einer Spritze oder einem Dosierbehälter, einem medizinischen Gerät oder dergleichen, was in der Regel nach der Kanülierung durchgeführt wird, die Gefahr besteht, dass das Gefäß mehr als notwendig belastet oder sogar beschädigt wird.

Ferner ist es für den Patienten wichtig, dass die fluide Verbindung zwischen der Kanüle und der mit der Kanüle verbundenen Komponente sowie insbesondere im Falle eines externen Fluidsystems sämtliche Fluidverbindungen des Fluidsystem sicher geschlossen sind, denn das unbeabsichtigte Lösen der Fluidverbindung aufgrund einer nicht richtig hergestellten Verbindung, beispielsweise zwischen einer Kanüle und einem Infusionsschlauch, kann lebensbedrohliche Folgen haben.

Aus den vorgenannten Gründen erscheint es vorteilhaft, eine Vorrichtung bereitzustellen, mit der medizinische Fluidverbindungen, insbesondere Luer-Lock-Verbindungen, automatisiert und autonom herstellbar sind.

Die Herstellung einer Luer-Lockverbindung mithilfe einer Vorrichtung ist aus dem Stand der Technik grundsätzlich bekannt, insbesondere aus der WO 2004/047883 A2. Allerdings handelt es sich bei der in der WO 2004/047883 A2 beschriebenen Vorrichtung um eine manuell betätigbare Vorrichtung, insbesondere um eine in Verbindung mit einem sogenannten "Autoinjektor" verwendbare Vorrichtung. Autoinjektoren sind Vorrichtungen, welche insbesondere zur eigenständigen Injektion durch den Patienten selbst eingesetzt werden, beispielsweise bei der Insulingabe durch Diabetespatienten oder zur Notfallinjektion von Adrenalin bei einem allergischen Schock.

Der vorliegenden Erfindung liegt die Aufgabe zugrunde, eine verbesserte Vorrichtung zum Herstellen und/oder Lösen einer mit einem Fluid durchströmbaren, medizinischen Gewindeverbindung aus einem ersten Verbindungselement und einem zweiten Verbindungselement bereitzustellen, insbesondere eine Vorrichtung, mittels welcher eine mit einem Fluid durchströmbare medizinische Gewindeverbindung automatisiert hergestellt und/oder gelöst werden kann.

Zur Lösung dieser Aufgabe dienen eine Vorrichtung zum Herstellen und/oder Lösen einer mit einem Fluid durchströmbaren, medizinischen Gewindeverbindung gemäß Patentanspruch 1 sowie ein Verfahren zum Herstellen und/oder Lösen einer mit einem Fluid durchströmbaren, medizinischen Gewindeverbindung gemäß Patentanspruch 16. Bevorzugte Ausgestaltungen der Erfindung sind Gegenstand der Unteransprüche und der vorliegenden Beschreibung der Erfindung.

Eine **erfindungsgemäße Vorrichtung** zum Herstellen und/oder Lösen einer mit einem Fluid durchströmbaren, medizinischen Gewindeverbindung aus einem ersten Verbindungselement und einem zweiten Verbindungselement, insbesondere zum Herstellen und/oder Lösen einer Luer-Lock-Verbindung, weist eine erste Aufnahmeeinrichtung zur Aufnahme des ersten Verbindungselements und eine zweite Aufnahmeeinrichtung zur Aufnahme des zweiten Verbindungselements auf, wobei die erste Aufnahmeeinrichtung und die zweite Aufnahmeeinrichtung um eine gemeinsame Verschraubungsachse relativ zueinander drehbar sind und entlang der Verschraubungsachse relativ zueinander verlagerbar sind. Dabei ist die Vorrichtung dazu eingerichtet, nach dem Einbringen des ersten Verbindungselementes in die erste Aufnahmeeinrichtung und dem Einbringen des zweiten Verbindungselementes in die zweite Aufnahmeeinrichtung, die erste Aufnahmeeinrichtung und die zweite Aufnahmeeinrichtung derart relativ zueinander zu bewegen, dass das erste Verbindungselement und das zweite Verbindungselement miteinander verschraubt werden oder die Gewindeverbindung gelöst wird.

Ein Merkmal einer erfindungsgemäßen Vorrichtung ist insbesondere, dass in die erste Aufnahmeeinrichtung und/oder in die zweite Aufnahmeeinrichtung das zugehörige Verbindungselement bezogen auf die Verschraubungsachse in radialer Richtung einbringbar ist.

Dadurch, dass bei einer erfindungsgemäßen Vorrichtung zum Herstellen und/oder Lösen einer medizinischen Gewindeverbindung wenigstens ein zum Herstellen der Gewindeverbindung erforderliches Verbindungselement, bezogen auf die Verschraubungsachse, in radialer Richtung in die jeweils zugehörige Aufnahmeeinrichtung eingebracht werden kann, können mit einer erfindungsgemäßen Vorrichtung auf besonders einfache Art und Weise auch medizinische Gewindeverbindungen aus einem ersten Verbindungselement und einem zweiten Verbindungselement hergestellt werden, bei welchem wenigstens eines der Verbindungselemente einen Schlauch aufweist oder mit einem Schlauch verbunden ist, denn das Einbringen wenigstens eines Verbindungselementes in radialer Richtung ist insbesondere für Verbindungselemente, die einen Schlauch aufweisen oder mit einem Schlauch verbunden sind, deutlich einfacher und damit auch deutlich vorteilhafter als ein axiales Einbringen.

Insbesondere können mittels einer erfindungsgemäßen Vorrichtung sämtliche Gewindeverbindungen eines externen Fluidsystems sicher und reproduzierbar hergestellt werden.

Eine Gewindeverbindung im Sinne der Erfindung ist dabei eine Verbindung, welche durch Verschrauben eines Innengewindes mit einem Außengewinde hergestellt ist.

Eine medizinische Gewindeverbindung ist dabei eine zur Verwendung in einem medizinischen Bereich geeignete Gewindeverbindung, insbesondere eine Gewindeverbindung, welche aus einem sterilisierbaren Material hergestellt ist. Eine mit einem Fluid durchströmbare Gewindeverbindung ist eine Gewindeverbindung, durch welche ein Fluid strömen kann, im Fall einer medizinischen Gewindeverbindung insbesondere ein als Fluid vorliegendes Medikament und/oder eine Körperflüssigkeit und/oder ein Gas, wobei die Gewindeverbindung in diesem Fall vorzugsweise gasdicht und/oder flüssigkeitsdicht ausgebildet ist.

Als Verschraubungsachse im Sinne der Erfindung wird dabei diejenige Achse bezeichnet, um welche das erste Verbindungselement und das zweite Verbindungselement zum Herstellen und/oder Lösen der Gewindeverbindung relativ zueinander zu drehen sind.

Vorzugsweise ist eine erfindungsgemäße Vorrichtung dabei derart ausgebildet, dass das erste Verbindungselement und/oder das zweite Verbindungselement in die jeweils zugehörige Aufnahmeeinrichtung in radialer Richtung, bezogen auf die Verschraubungsachse, eingelegt werden können. In einigen Fällen kann es aber auch vorteilhaft sein, wenn das erste Verbindungselement und/oder das zweite Verbindungselement in die jeweils zugehörige Aufnahmeeinrichtung in radialer Richtung eingeschoben werden kann, insbesondere eingeführt werden kann.

Vorzugsweise ist eine erfindungsgemäße Vorrichtung ferner derart ausgebildet, dass das erste Verbindungselement und/oder das zweite Verbindungselement manuell und/oder automatisiert in die jeweils zugehörige Aufnahmeeinrichtung eingebracht werden kann. Zum automatisierten Einbringen des ersten Verbindungselementes und/oder des zweiten Verbindungselementes in die Vorrichtung, insbesondere in die erste Aufnahmeeinrichtung und/oder in die zweite Aufnahmeeinrichtung, ist die Vorrichtung vorzugsweise derart ausgestaltet, dass das erste Verbindungselement und/oder das zweite Verbindungselement zum Einbringen entlang einer möglichst einfachen Bahnkurve geführt werden kann.

Die beiden Aufnahmeeinrichtungen einer erfindungsgemäßen Vorrichtung, das heißt die erste Aufnahmeeinrichtung und die zweite Aufnahmeeinrichtung, sind vorzugsweise translatorisch in axialer Richtung entlang der gemeinsamen Verschraubungsachse relativ zueinander bewegbar und insbesondere relativ zueinander um die Verschraubungsachse drehbar.

Besonders bevorzugt können die erste Aufnahmeeinrichtung und die zweite Aufnahmeeinrichtung zum Herstellen der Gewindeverbindung zunächst in axialer Richtung zusammengeführt, insbesondere zusammengedrückt, werden, vorzugsweise wenigstens in Kontakt gebracht werden, um dann anschließend um die Verschraubungsachse herum relativ zueinander gedreht werden zu können, insbesondere unter Aufbringen einer definierten Axialkraft, insbesondere unter Aufbringen einer zusammendrückend wirkenden Axialkraft.

Zum Lösen einer Gewindeverbindung können die erste Aufnahmeeinrichtung und die zweite Aufnahmeeinrichtung vorzugsweise in einem ersten Schritt zunächst um die Verschraubungsachse relativ zueinander verdreht werden, insbesondere unter einer auseinanderziehend wirkenden Axialkraft, um dann in einem weiteren Schritt translatorisch in axialer Richtung entlang der Verschraubungsachse relativ zueinander, insbesondere auseinander, bewegt werden zu können.

Bevorzugt ist eine erfindungsgemäße Vorrichtung derart ausgebildet, dass sowohl in die erste Aufnahmeeinrichtung und in die zweite Aufnahmeeinrichtung das jeweils zugehörige Verbindungselement, bezogen auf die Verschraubungsachse, in radialer Richtung eingebracht werden kann. Dadurch können auf besonders einfache Art und Weise Gewindeverbindungen mit Hilfe einer erfindungsgemäßen Vorrichtung hergestellt werden, bei welchen beide Verbindungselemente jeweils einen Schlauch aufweisen oder mit einem Schlauch verbunden sind.

Erfindungsgemäß weist dazu die erste Aufnahmeeinrichtung und/oder die zweite Aufnahmeeinrichtung eine zur Aufnahme des zugehörigen Verbindungselementes ausgebildete, in radialer Richtung nach außen hin geöffnete Ausnehmung oder einen Greifer auf. Das heißt, alternativ, insbesondere anstatt einer Aufnahmeeinrichtung mit einer in radialer Richtung nach außen hin geöffneten Ausnehmung, kann wenigstens eine Aufnahmeeinrichtung einer erfindungsgemäßen Vorrichtung auch einen Greifer aufweisen oder als Greifer ausgebildet sein.

Mit einer in radialer Richtung nach außen hin geöffneten Ausnehmung, ebenfalls bezogen auf die Verschraubungsachse, lässt sich jedoch auf besonders einfache Art und Weise, insbesondere ohne einen großen Steuerungsaufwand, eine Aufnahmeeinrichtung bereitstellen, in welche ein zugehöriges Verbindungselement in radialer Richtung eingebracht werden kann.

Um möglichst viele verschiedene Verbindungselemente aufnehmen zu können und somit zum Herstellen und/oder Lösen möglichst vieler verschiedener Gewindeverbindungen eingesetzt werden zu können, weist die erste Aufnahmeeinrichtung und/oder die zweite Aufnahmeeinrichtung einer erfindungsgemäßen Vorrichtung vorzugsweise einen oder mehrere auswechselbare Aufnahmeeinsätze und/oder einen oder mehrere auswechselbare Greifer auf. Dadurch kann auf einfache Art und Weise die Flexibilität der Vorrichtung erheblich erhöht werden.

In einer vorteilhaften Ausgestaltung einer erfindungsgemäßen Vorrichtung ist die erste Aufnahmeeinrichtung und/oder die zweite Aufnahmeeinrichtung dazu ausgebildet, das zugehörige Verbindungselement verdrehsicher in Bezug auf eine Drehung um die Verschraubungsachse aufzunehmen. Das heißt mit anderen Worten, in einer vorteilhaften Ausgestaltung einer erfindungsgemäßen Vorrichtung ist wenigstens eine der beiden Aufnahmeeinrichtungen dazu ausgebildet, das zugehörige Verbindungselement gesichert gegen ein Verdrehen um die Verschraubungsachse aufzunehmen. Vorzugsweise ist die erste Aufnahmeeinrichtung und/oder die zweite Aufnahmeeinrichtung dazu ausgebildet, ein zugehöriges, in die Aufnahmeeinrichtung eingelegtes Verbindungselement mittels eines Formschlusses, also formschlüssig, gegen ein Verdrehen zu sichern.

Dies kann beispielsweise erreicht werden, indem die Aufnahmeeinrichtung zumindest in einem Bereich des Verbindungselementes eine an die Außenkontur des Verbindungselementes angepasste Innenkontur aufweist, welche derart ausgebildet ist, dass ein Verdrehen um die Verschraubungsachse verhindert wird.

Weist ein aufzunehmendes Verbindungselement beispielsweise eine AußenSechskant-Kontur auf, kann mittels einer korrespondierend ausgebildeten InnenSechskant-Kontur auf einfache Art und Weise eine formschlüssige Verdrehsicherung geschaffen werden. Ebenso können weitere Geometrien eines aufzunehmenden Verbindungselementes zur Herstellung einer formschlüssigen Verdrehsicherung genutzt werden, beispielsweise die bei Luer-Lock-Verbindungselementen mit weiblichen Verbindungsabschnitten häufig vorhandenen sogenannten "Flügelchen" oder in axialer Richtung verlaufende und in radialer Richtung nach außen hervorstehende, am Verbindungselement angeordnete Rippen, mit welchen beispielsweise in Verbindung mit einem sich in radialer Richtung in die Ausnehmung nach innen hineinragenden Stift oder Steg ein gegen Verdrehen sichernder Formschluss herstellbar ist.

Alternativ oder zusätzlich kann die Aufnahmeeinrichtung auch ein Verdrehsicherungselement aufweisen, insbesondere ein aktives oder passives Verdrehsicherungselement, mittels dem zumindest nach dem Einbringen des Verbindungselementes in die Aufnahmeeinrichtung das Verbindungselement gegen ein Verdrehen um die Verschraubungsachse gesichert werden kann.

Dies kann beispielsweise mit Hilfe eines sich in radialer Richtung nach innen in die Aufnahmeeinrichtung erstreckenden Verdrehsicherungsstiftes oder Verdrehsicherungspins erreicht werden, wobei das Verdrehsicherungselement auch als aktives, vorzugsweise in radialer Richtung nach innen versenkbares Verdrehsicherungselement ausgebildet sein kann und erst nach dem Einbringen des Verbindungselementes mit dem eingebrachten Verbindungselement wirkverbunden werden kann.

Alternativ oder zusätzlich kann das Verbindungselement in der Aufnahmeeinrichtung auch kraftschlüssig bzw. reibschlüssig gegen ein Verdrehen gesichert werden, wobei dazu vorzugsweise wenigstens eine der Aufnahmeeinrichtungen eine Spanneinrichtung, aufweist, insbesondere eine spannbare Klemmeinrichtung.

Bei einer erfindungsgemäßen Vorrichtung sind vorzugsweise das erste Verbindungselement und das zweite Verbindungselement jeweils in axialer Richtung in der zugehörigen Aufnahmeeinrichtung festlegbar.

In einer weiteren vorteilhaften Ausgestaltung einer erfindungsgemäßen Vorrichtung ist die Vorrichtung zum zumindest teilweite automatisieren Herstellen und/oder Lösen der Gewindeverbindung eingerichtet. Dabei ist eine erfindungsgemäße Vorrichtung insbesondere zum automatisierten Verschrauben der beiden Verbindungselemente miteinander und/oder zum automatisierten Lösen der Verschraubung der Gewindeverbindung eingerichtet, wobei die Vorrichtung dazu vorzugsweise eine Steuerungseinrichtung aufweist, insbesondere eine Steuerungseinrichtung mit einer Sensoreinrichtung und/oder einer Aktuatoreinrichtung. Die Steuereinrichtung ist vorzugsweise Teil eines Kanülierautomaten, insbesondere Teil der Steuereinrichtung des Kanülierautomaten, mittels der insbesondere die automatische Punktion des Blutgefäßes enies Patienten gesteuert wird.

Besonders bevorzugt ist eine erfindungsgemäße Vorrichtung dazu ausgebildet, in Verbindung mit einem sogenannten Kanülierautomat, der wenigstens eine Aufnahmeeinrichtung zur Aufnahme einer Kanüle aufweist, zusammen verwendet zu werden, wobei vorzugsweise die Aufnahmeeinrichtung des Kanülierautomaten, mittels welcher die Kanüle aufgenommen, gehalten und/oder beim Kanülieren geführt wird, eine Aufnahmeeinrichtung der erfindungsgemäßen Vorrichtung bildet, insbesondere die erste Aufnahmeeinrichtung.

Kanülierautomaten sind aus dem Stand der Technik grundsätzlich bekannt, beispielsweise aus der US 2015/0065916 A1, der WO 2015/052719 A1 und der WO 2010/029521 A2, auf welche hiermit für nähere Ausführungen zu Kanülierautomaten verwiesen wird. Kanülierautomaten sind in der Regel dazu ausgebildet, einen Kanüliervorgang am Patienten autonom vorzubereiten und die Kanülierung mit Hilfe geeigneter Sensorik und Aktuatorik autonom durchzuführen.

Vorzugsweise ist eine erfindungsgemäße Vorrichtung dabei dazu eingerichtet, eine Gewindeverbindung aus einem ersten Verbindungselement und einem zweiten Verbindungselement nach einem Kanüliervorgang herzustellen, wobei mittels einer erfindungsgemäßen Vorrichtung vorzugsweise die Kanüle als erstes Verbindungselement mit einem zweiten Verbindungselement, vorzugsweise einem Schlauchanschluss oder dergleichen, verschraubt werden kann.

Ist eine erfindungsgemäße Vorrichtung zum Herstellen einer Gewindeverbindung zwischen einer Kanüle und einem weiteren Verbindungselement nach dem Kanüliervorgang ausgebildet, kann die Gewindeverbindung vorzugsweise unmittelbar nach dem Kanüliervorgang hergestellt werden, insbesondere vor dem Freigeben der Kanüle durch die Aufnahmeeinrichtung des Kanülierautomaten, wobei in diesem Fall vorzugsweise die Aufnahmeeinrichtung des Kanülierautomaten, mit welchem die Kanüle gehalten wird, als Aufnahmeeinrichtung, insbesondere als erste Aufnahmeeinrichtung, verwendet wird. Dadurch kann ein Mehrfach-Handling der bereits im Patienten befindlichen Kanüle vermieden werden und somit eine mechanische Belastung des mittels der Kanüle punktierten Gefäßes so gering wie möglich gehalten werden.

Andererseits ist eine erfindungsgemäße Vorrichtung ferner vorzugsweise dazu eingerichtet, eine Gewindeverbindung unabhängig von einem Kanüliervorgang herzustellen und/oder zu lösen. Insbesondere ist eine erfindungsgemäße Vorrichtung vorzugsweise dazu eingerichtet, eine oder mehrere Gewindeverbindungen eines externen, mit einem Patienten fluidkommunizierend verbindbaren Fluidsystems unabhängig von einem Kanüliervorgang, insbesondere im Vorfeld, das heißt nicht am Patienten, herzustellen und/oder zu lösen.

Eine erfindungsgemäße Vorrichtung ermöglicht somit auf besonders einfache Art und Weise die Herstellung und/oder das Lösen von Gewindeverbindungen eines Fluidsystems, insbesondere von medizinischen Gewindeverbindungen, insbesondere von Schlauchverbindungen, wobei die Gewindeverbindungen automatisiert und damit besonders sicher und reproduzierbar hergestellt und/oder gelöst werden können, und insbesondere weitgehend beschädigungsfrei.

In einigen Fällen kann es vorteilhaft sein, wenn nicht nur das Verschrauben als solches mit einer erfindungsgemäßen Vorrichtung automatisiert möglich ist und/oder das Lösen der Gewindeverbindung als solches, sondern wenn außerdem auch das Einbringen des ersten Verbindungselementes und/oder des zweiten Verbindungselementes und/oder das Bereitstellen des ersten Verbindungselementes und/oder des zweiten Verbindungselementes automatisiert möglich sind.

Weist eine erfindungsgemäße Vorrichtung eine Sensorvorrichtung auf, handelt es sich vorzugsweise um eine Drehmomentsensorvorrichtung und/oder eine Axialkraftmessvorrichtung.

Mit Hilfe einer Sensorvorrichtung ist eine besonders präzise Steuerung einer erfindungsgemäßen Vorrichtung möglich, und infolgedessen können Gewindeverbindungen besonders reproduzierbar hergestellt und/oder gelöst werden, insbesondere lassen sich auf diese Weise besonders zuverlässig sichere Gewindeverbindungen herstellen, da insbesondere eine Beschädigung der Gewindeverbindung durch Aufbringen eines zu hohen Drehmoments und/oder einer zu hohen Axialkraft auf diese Weise einfach ermittelt werden können.

Alternativ oder zusätzlich kann bei einer erfindungsgemäßen Vorrichtung anstatt einer vorbeschriebenen kraft- und/oder drehmomentgesteuerten Herstellung der Gewindeverbindung bzw. einem kraft- und/oder drehmomentgesteuerte Lösen der Gewindeverbindung die Gewindeverbindung auch drehwinkelgesteuert und/oder weggesteuert hergestellt und/oder gelöst werden, insbesondere geregelt.

Dazu weist eine erfindungsgemäße Vorrichtung vorzugsweise eine Winkelmessvorrichtung zur Erfassung des zurückgelegten Relativwinkels und/oder eine Wegmessvorrichtung zur Erfassung der axial entlang der Verschraubungsachse zurückgelegten Wegstrecke der ersten Aufnahmeeinrichtung und/oder der zweiten Aufnahmeeinrichtung, insbesondere zur Erfassung der relativ zurückgelegten Wegstrecke, auf.

Mittels einer geeignet ausgebildeten Sensorvorrichtung und einer entsprechend ausgebildeten Steuerungseinrichtung können Gewindeverbindungen nicht nur reproduzierbar gelöst oder hergestellt werden, sondern auf besonders einfache Art und Weise kann auch die Herstellung und/oder das Lösen der Gewindeverbindung überwacht werden. Insbesondere kann auf diese Art und Weise festgestellt werden, ob das erste Verbindungselement und das zweite Verbindungselement richtig miteinander verschraubt sind oder die Gewindeverbindung vollständig gelöst ist, wobei dazu vorzugsweise eine Sollwert-Vorgabe mit einer Istwert-Vorgabe verglichen wird, insbesondere ein Soll-Drehmoment mit einem Ist-Drehmoment-Wert.

Zum Schutz vor einer Beschädigung der Gewindeverbindung ist es außerdem vorteilhaft, wenn die Vorrichtung dazu ausgebildet ist, bei Überschreiten eines vorgegebenen Schwellwertes die Relativbewegung zwischen der ersten Aufnahmeeinrichtung und der zweiten Aufnahmeeinrichtung anzuhalten. Alternativ kann auch eine Soll-Stromkurve eines Antriebsmotors erfasst werden und insbesondere mit einer Ist-Stromkurve verglichen werden und insbesondere bei Überschreiten eines definierten Schwellwertes der Antriebsmotor abgeschaltet werden. Dadurch kann auf eine besonders einfache Art und Weise eine Überlastsicherung zum Schutz der Gewindeverbindung bereitgestellt werden und besonders einfach und effektiv eine Beschädigung der Gewindeverbindung vermieden werden.

Eine alternative Möglichkeit zur Realisierung eines Überlastschutzes und/oder zur Bereitstellung eines Toleranzausgleiches besteht darin, bei einer erfindungsgemäßen Vorrichtung ein Federelement und/oder ein Dämpferelement vorzusehen, welches vorzugsweise in einem Bypass-Lastpfad angeordnet ist, wobei eine erfindungsgemäße Vorrichtung in diesem Fall vorzugsweise dazu ausgebildet ist, nach Überschreiten einer definierten, maximal zulässigen Axialkraft und/oder nach Überschreiten eines definierten Drehmomentes, eine (weiterhin) erzeugte Axialkraft und/oder ein (weiterhin) erzeugtes Drehmoment über den Bypass-Lastpfad auf das Federelement und/oder das Dämpferelement abzuleiten und nicht weiter auf die Gewindeverbindung zu übertragen, wodurch die Gewindeverbindung vor einer Überlastung, insbesondere einer mechanischen Überlastung, geschützt werden kann.

In einer weiteren vorteilhaften Ausgestaltung einer erfindungsgemäßen Vorrichtung ist eine der beiden Aufnahmeeinrichtungen, insbesondere die erste Aufnahmeeinrichtung, zur Aufnahme eines ersten Verbindungselementes mit einem ersten Verbindungsabschnitt mit einem konzentrisch zu einer ersten Achse angeordneten hülsenförmigen Innengewinde ausgebildet, insbesondere zur Aufnahme eines ersten Verbindungselementes mit einem männlichen Luer-Lock-Verbindungsabschnitt, während die andere Aufnahmeeinrichtung, insbesondere die zweite Aufnahmeeinrichtung, zur Aufnahme eines zweiten Verbindungselements mit einem zweiten Verbindungsabschnitt mit einem konzentrisch zu einer zweiten Achse angeordneten Außengewinde ausgebildet ist, insbesondere zur Aufnahme eines zweiten Verbindungselementes mit einem weiblichen Luer-Lock-Verbindungsabschnitt.

Aufgrund der Tatsache, dass üblicherweise Verbindungselemente mit einem Luer-Lock-Verbindungsabschnitt eingesetzt werden, insbesondere im medizinischen Bereich, ergibt sich für eine entsprechend ausgestaltete erfindungsgemäße Vorrichtung somit ein breites Einsatzgebiet.

In einer weiteren vorteilhaften Ausgestaltung einer erfindungsgemäßen Vorrichtung ist die Vorrichtung derart ausgebildet, dass nach dem Einbringen des ersten Verbindungselementes in die erste Aufnahmeeinrichtung und nach dem Einbringen des zweiten Verbindungselementes in die zweite Aufnahmeeinrichtung die erste Achse des ersten Verbindungselementes und die zweite Achse des zweiten Verbindungselementes entlang der Verschraubungsachse ausgerichtet sind oder entlang der Verschraubungsachse ausrichtbar sind. Dadurch kann erreicht werden, dass insbesondere die erste Aufnahmeeinrichtung und die zweite Aufnahmeeinrichtung bzw. die in diesen jeweils aufgenommenen Verbindungselemente, zueinander ausgerichtet sind, insbesondere derart, dass die erste Achse des ersten Verbindungselementes mit der zweiten Achse des zweiten Verbindungselementes zusammenfällt und die erste Achse des ersten Verbindungselementes und die zweite Achse des zweiten Verbindungselementes dabei vorzugsweise mit der Verschraubungsachse zusammenfallen.

Eine derartige Ausrichtung ist Voraussetzung für eine sichere und reproduzierbare Herstellung einer Gewindeverbindung und/oder zum sicheren und insbesondere beschädigungsfreien Lösen einer Gewindeverbindung. Die Vorrichtung, insbesondere die erste Aufnahmeeinrichtung und die zweite Aufnahmeeinrichtung, kann dabei entweder derart ausgebildet sein, dass die erste Aufnahmeeinrichtung und die zweite Aufnahmeeinrichtung stets entsprechend zueinander ausgerichtet sind, so dass bereits das Einbringen des ersten Verbindungselementes in die erste Aufnahmeeinrichtung und das Einbringen des zweiten Verbindungselementes in die zweite Aufnahmeeinrichtung bewirkt, dass das erste Verbindungselement und das zweite Verbindungselement derart zueinander angeordnet und ausgerichtet sind und die erste Achse des ersten Verbindungselementes und die zweite Achse des zweiten Verbindungselementes jeweils zusammenfallen und insbesondere mit der Verschraubungsachse zusammenfallen.

Ist dies nicht der Fall, ist eine erfindungsgemäße Vorrichtung vorzugsweise derart ausgebildet, dass vor dem eigentlichen Verschrauben des ersten Verbindungselementes mit dem zweiten Verbindungselement bzw. vor dem Lösen der Gewindeverbindung, die erste Aufnahmeeinrichtung und die zweite Aufnahmeeinrichtung entsprechend zueinander ausgerichtet werden können, dass das erste Verbindungselement und das zweite Verbindungselement vor dem eigentlichen Verschrauben bzw. dem Lösen der Gewindeverbindung wie vorstehend beschrieben zueinander ausgerichtet sind.

In einer weiteren vorteilhaften Ausgestaltung einer erfindungsgemäßen Vorrichtung ist eine der beiden Aufnahmeeinrichtungen, insbesondere die erste Aufnahmeeinrichtung, ortsfest angeordnet und die andere Aufnahmeeinrichtung, insbesondere die zweite Aufnahmeeinrichtung, in axialer Richtung entlang der Verschraubungsachse bewegbar und um die Verschraubungsachse drehbar. Dadurch kann eine besonders einfache Vorrichtung bereitgestellt werden, da lediglich die zweite Aufnahmeeinrichtung entsprechend bewegbar gelagert sein muss und nur die Bewegungen der zweiten Aufnahmeeinrichtung gesteuert bzw. geregelt werden müssen.

Vorzugsweise ist wenigstens eine Aufnahmeeinrichtung dabei als feste Einspannung ausgebildet, insbesondere die erste Aufnahmeeinrichtung, während die andere Aufnahmeeinrichtung vorzugsweise wenigstens entlang der Verschraubungsachse translatorisch bewegbar ist und um die Verschraubungsachse drehbar.

Erfindungsgemäß zum automatisierten Herstellen und/oder Lösen einer Gewindeverbindung, weist eine erfindungsgemäße Vorrichtung eine Aktuator-Einrichtung zur Erzeugung der Relativbewegung zwischen der ersten Aufnahmeeinrichtung und der zweiten Aufnahmeeinrichtung auf.

In einer weiteren vorteilhaften Ausgestaltung einer erfindungsgemäßen Vorrichtung weist die Vorrichtung einen Spindelantrieb mit einer mittels eines Antriebsmotors antreibbaren Gewindespindel zur Erzeugung der Relativbewegung zwischen der ersten Aufnahmeeinrichtung und der zweiten Aufnahmeeinrichtung auf, wobei die Gewindespindel vorzugsweise mittels wenigstens eines ortsfest angeordneten Lagers geführt ist. Dabei weist der Spindelantrieb vorzugsweise einen ersten Antriebsmotor und eine mittels des ersten Antriebsmotors antreibbare und um die Verschraubungsachse drehbare Gewindespindel auf.

Ist die Gewindespindel dabei selbsthemmend ausgebildet, ist vorzugsweise eine Drehbewegung der Gewindespindel um die Verschraubungsachse mittels des Antriebsmotors bewirkbar, wobei der Antriebsmotor dazu vorzugsweise mit der Gewindespindel drehverbunden oder drehverbindbar ist, falls erforderlich mit einem Ubersetzungsgetriebe dazwischen, wobei der Antriebsmotor vorzugsweise dazu ausgebildet ist, die Gewindespindel um die Verschraubungsachse zu drehen.

Ist die Gewindespindel hingegen als nicht selbsthemmend ausgebildet, ist der Antriebsmotor vorzugsweise dazu ausgebildet, eine Axialkraft auf die Gewindespindel aufzubringen, vorzugsweise mittels eines Zahnstangengetriebes oder hydraulisch oder pneumatisch, wobei im Fall eines Zahnstangengetriebes der Antriebsmotor vorzugsweise mit einem Ritzel eines Zahnstangengetriebes drehverbunden oder drehverbindbar ist und die Zahnstange mit der Gewindespindel wirkverbunden ist und insbesondere derart zur Verschraubungsachse bewegbar ist, dass durch eine Linearbewegung der Zahnstange entlang der Verschraubungsachse, welche mit Hilfe des Antriebsmotors über das Ritzel bewirkbar ist, eine Axialbewegung der Gewindespindel entlang der Verschraubungsachse bewirkbar ist.

Aufgrund der nicht selbsthemmend ausgebildeten Gewindespindel führt die Axialbewegung in diesem Fall zu einer Drehbewegung der Gewindespindel und infolgedessen insbesondere zu einer axialen Verlagerung der Gewindespindel entlang der Verschraubungsachse.

Die Gewindespindel weist dabei vorzugsweise ein Außengewinde auf und das wenigstens eine ortsfest angeordnete Lager ist insbesondere zumindest teilweise ein entsprechend ausgebildetes Innengewinde. Aufgrund der ortsfesten Anordnung des Lagers führt die mit Hilfe des Antriebsmotors erzeugte Axialbewegung der Gewindespindel bei einem nicht selbsthemmenden Spindelantrieb zu einer Drehbewegung der Gewindespindel um die Verschraubungsachse.

Ist hingegen die Gewindespindel bzw. der Spindelantrieb selbsthemmend ausgebildet bzw. selbsthemmend in dem ortsfest angeordneten Lager geführt, wird eine auf die Gewindespindel aufgebrachte Axialkraft aufgrund der Selbsthemmung vom ortsfest angeordneten Lager abgestützt. In diesem Fall kann eine Axialbewegung der Gewindespindel nur durch ein Drehen der Gewindespindel um die Verschraubungsachse bewirkt werden, so dass bei einer selbsthemmend ausgebildeten Gewindespindel die Gewindespindel vorzugsweise mit Hilfe des Antriebsmotors um die Verschraubungsachse drehbar ist.

In einer weiteren vorteilhaften Ausgestaltung einer erfindungsgemäßen Vorrichtung ist die erste Aufnahmeeinrichtung oder die zweite Aufnahmeeinrichtung, insbesondere die zweite Aufnahmeeinrichtung, mit der Gewindespindel drehverbunden oder drehverbindbar, wobei die Aufnahmeeinrichtung vorzugsweise in einer radialen Ausnehmung der Gewindespindel angeordnet ist oder durch eine radiale Ausnehmung in der Gewindespindel gebildet ist. Dadurch lässt sich auf besonders einfache Art und Weise die zum Herstellen und/oder Lösen der Gewindeverbindung erforderliche Relativbewegung zwischen der ersten Aufnahmeeinrichtung und der zweiten Aufnahmeeinrichtung erzeugen.

Dabei ist vorzugsweise nur eine der Aufnahmeeinrichtungen mit der Gewindespindel drehverbunden oder drehverbindbar bzw. durch eine radiale Aufnahme in der Gewindespindel gebildet oder in einer radialen Ausnehmung der Gewindespindel angeordnet. Es können jedoch beide Aufnahmeeinrichtungen jeweils mit einer Gewindespindel drehverbunden oder drehverbindbar sein und vorzugsweise in einer radialen Ausnehmung der Gewindespindel angeordnet oder durch eine radiale Ausnehmung der Gewindespindel gebildet sein. Dadurch können auf besonders einfache Art und Weise beide Aufnahmeeinrichtungen separat voneinander bewegt werden.

Besonders bevorzugt ist jedoch die eine der beiden Aufnahmeeinrichtungen ortsfest ausgebildet, insbesondere die erste Aufnahmeeinrichtung, und lediglich die zweite Aufnahmeeinrichtung mit einer Gewindespindel drehverbunden oder drehverbindbar und vorzugsweise in einer radialen Ausnehmung der Gewindespindel angeordnet oder durch eine radiale Ausnehmung in der Gewindespindel gebildet.

In einer alternativen Ausgestaltung einer erfindungsgemäßen Vorrichtung weist die Vorrichtung zur Erzeugung der Relativbewegung zwischen der ersten Aufnahmeeinrichtung und der zweiten Aufnahmeeinrichtung einen ersten Antriebsmotor und ein mittels des ersten Antriebsmotors antreibbares Getriebe mit einem ersten Getriebeelement und wenigstens einem zweiten, mit dem ersten Getriebeelement drehverbunden zweiten Getriebeelement auf, wobei die erste Aufnahmeeinrichtung oder die zweite Aufnahmeeinrichtung, insbesondere die zweite Aufnahmeeinrichtung mit dem ersten Getriebeelement drehverbunden oder drehverbindbar ist. Auf diese Weise kann ebenfalls auf besonders einfache Art und Weise eine Relativbewegung zwischen der ersten Aufnahmeeinrichtung und der zweiten Aufnahmeeinrichtung bewirkt werden.

Vorzugsweise ist das Getriebe dabei ein Zahnradgetriebe, insbesondere ein Stirnradgetriebe, wobei das Getriebe insbesondere mit dem Antriebsmotor drehverbunden oder drehverbindbar ist. Alternativ kann das Getriebe auch ein Riemengetriebe sein mit wenigstens zwei Riemenscheiben, welche über einen Riemen miteinander wirkverbunden sind, oder ein Reibradgetriebe mit wenigstens zwei Reibrädern, welche reibschlüssig miteinander wirkverbunden sind.

Ist das Getriebe als Zahnradgetriebe ausgebildet, ist das erste Getriebeelement vorzugsweise ein Zahnrad und das zweite Getriebeelement ein Zahnrad, wobei das zweite Zahnrad vorzugsweise mit dem ersten Zahnrad kämmt. Besonders bevorzugt ist das Getriebe dabei mittels des ersten Antriebsmotors antreibbar, insbesondere über das zweite Getriebeelement.

In einer weiteren vorteilhaften Ausgestaltung einer erfindungsgemäßen Vorrichtung ist die erste Aufnahmeeinrichtung oder die zweite Aufnahmeeinrichtung, insbesondere die zweite Aufnahmeeinrichtung, in einer radialen Ausnehmung des ersten Getriebeelementes angeordnet oder durch eine radiale Ausnehmung im ersten Getriebeelement gebildet. Dadurch kann auf besonders einfache Art und Weise die erste Aufnahmeeinrichtung um die Verschraubungsachse zum Herstellen und/oder Lösen der Gewindeverbindung gedreht werden.

In einigen Fällen, insbesondere wenn zum Verschrauben ein Drehwinkel von mehr als 360° minus dem Öffnungswinkel der Ausnehmung im ersten Getriebeelement erforderlich ist, weist eine erfindungsgemäße Vorrichtung ein drittes, mit dem ersten Getriebeelement drehverbundenes oder drehverbindbares Getriebeelement auf, wobei das dritte Getriebeelement vorzugsweise mittels des ersten Antriebsmotors und/oder mittels eines weiteren Antriebsmotors antreibbar ist. Dadurch kann in dem Winkelbereich, in welchem das erste Getriebeelement aufgrund seiner Ausnehmung in radialer Richtung nicht mittels des zweiten Getriebeelementes antreibbar ist, das erste Getriebeelement mit Hilfe des dritten Getriebeelementes angetrieben werden und somit der durch die Ausnehmung im ersten Getriebeelement nicht zum Antrieb mittels des zweiten Getriebeelements nutzbare Winkelbereich mit Hilfe des dritten Getriebeelementes überbrückt werden.

Dies kann insbesondere erforderlich sein, wenn zum Herstellen einer sicheren Gewindeverbindung eine Relativdrehung um die Verschraubungsachse zwischen dem ersten Verbindungselement und dem zweiten Verbindungselement von mehr als 360° minus des Öffnungswinkels der Ausnehmung im ersten Getriebeelement erforderlich ist, was insbesondere dann der Fall sein kann, wenn wenigstens eines der Verbindungselemente in Umfangsrichtung nicht reproduzierbar eingelegt werden kann, so dass zum Herstellen der Gewindeverbindung und/oder zum Lösen der Gewindeverbindung die erforderlichen Drehwinkel stark schwanken.

In einer weiteren vorteilhaften Ausgestaltung einer erfindungsgemäßen Vorrichtung ist die Vorrichtung dazu eingerichtet, die erste Aufnahmeeinrichtung und die zweite Aufnahmeeinrichtung entlang der Verschraubungsachse zusammenzuschieben, insbesondere zusammenzudrücken, und/oder auseinanderzuziehen. Dazu weist die Vorrichtung, insbesondere die Aktuatoreinrichtung, einer erfindungsgemäßen Vorrichtung eine Axialkrafterzeugungseinrichtung auf. Dies ist insbesondere vorteilhaft bzw. in einigen Fällen sogar notwendig, wenn die Vorrichtung keinen Spindelantrieb aufweist, sondern zur Erzeugung der Relativbewegung zwischen der ersten Aufnahmeeinrichtung und der zweiten Aufnahmeeinrichtung lediglich ein herkömmliches, mittels eines Antriebsmotors antreibbares Getriebe aufweist.

Als Axialkrafterzeugungseinrichtung eignet sich insbesondere ein federvorgespanntes System, insbesondere mit wenigstens einer Schraubenfeder zur Erzeugung der erforderlichen Axialkraft, wobei die Feder vorzugsweise derart angeordnet ist, dass eine Axialkraft in die gewünschte Richtung entsteht, das heißt insbesondere zum Zusammendrücken und/oder zum Auseinanderschieben bzw. -ziehen der ersten Aufnahmeeinrichtung und der zweiten Aufnahmeeinrichtung, wobei es in einigen Fällen vorteilhaft sein kann, wenn die Vorrichtung zusätzlich eine Arretiereinrichtung aufweist, mittels der die erste Aufnahmeeinrichtung und/oder die zweite Aufnahmeeinrichtung entgegen der mittels der Feder erzeugten Axialkraft in axialer Richtung arretiert werden kann.

In einer weiteren alternativen Ausgestaltung einer erfindungsgemäßen Vorrichtung ist die erste Aufnahmeeinrichtung und/oder die zweite Aufnahmeeinrichtung, insbesondere die zweite Aufnahmeeinrichtung, an einem Greiferarm befestigt, wobei der Greiferarm vorzugsweise zumindest entlang der Verschraubungsachse verlagerbar und insbesondere um die Verschraubungsachse herum rotierbar ist. Besonders bevorzugt ist die erste Aufnahmeeinrichtung und/oder die zweite Aufnahmeeinrichtung, insbesondere die zweite Aufnahmeeinrichtung, durch einen Greifer gebildet.

In einer weiteren vorteilhaften Ausgestaltung einer erfindungsgemäßen Vorrichtung ist die Vorrichtung derart ausgebildet, dass das erste Verbindungselement und das zweite Verbindungselement der Gewindeverbindung nach dem Herstellen der Gewindeverbindung jeweils in radialer Richtung aus der ersten Aufnahmeeinrichtung und der zweiten Aufnahmeeinrichtung entnehmbar sind. Dazu weisen vorzugsweise beide Aufnahmeeinrichtungen jeweils eine zur Aufnahme des zugehörigen Verbindungselementes ausgebildete, in radialer Richtung nach außen hin geöffnete Ausnehmung auf oder jeweils einen Greifer.

Dabei befinden sich insbesondere nach dem Herstellen der Gewindeverbindung die radiale Ausnehmung der ersten Aufnahmeeinrichtung in einem ersten definierten Winkelbereich in Umfangsrichtung und die Ausnehmung der zweiten Aufnahmeeinrichtung in einem zweiten Winkelbereich, wobei sich der erste Winkelbereich und der zweite Winkelbereich zumindest teilweise überlappen. Eine derartig ausgestaltete Vorrichtung ermöglicht auf besonders einfache Art und Weise das Entnehmen und/oder Auswerfen einer mittels einer mit Hilfe einer erfindungsgemäßen Vorrichtung hergestellten Gewindeverbindung.

Insbesondere können mit einer derartig ausgebildeten, erfindungsgemäßen Vorrichtung Gewindeverbindung mit einem Schlauchelemente, insbesondere medizinische Luer-Lock-Gewindeverbindungen mit wenigstens einem Schlauch, besonders schnell und einfach automatisiert hergestellt. Insbesondere können bei einem externen Fluidsystem sämtliche Schlauchverbindungen mit einer erfindungsgemäßen Vorrichtung äußerst effizient und automatisiert und damit reproduzierbar und sicher hergestellt werden.

Der Winkelbereich, in welchem sich die radialen Ausnehmungen der ersten Aufnahmeeinrichtung und der zweiten Aufnahmeeinrichtung vorzugsweise nach dem Herstellen der Gewindeverbindung befinden, sind dabei insbesondere derart gewählt, dass die hergestellte Gewindeverbindung, falls gewünscht, durch die Schwerkraft herausfällt oder eben gerade nicht durch die Schwerkraft herausfällt und bis zum Entnehmen sicher in der Vorrichtung gehalten ist.

In einer weiteren vorteilhaften Ausgestaltung einer erfindungsgemäßen Vorrichtung ist die Vorrichtung derart ausgebildet, dass zum Lösen der Gewindeverbindung das erste Verbindungselement und das zweite Verbindungselement der Gewindeverbindung in miteinander verschraubtem Zustand jeweils in radialer Richtung in die erste Aufnahmeeinrichtung und die zweite Aufnahmeeinrichtung einbringbar sind. Dazu weisen vorzugsweise beide Aufnahmeeinrichtungen jeweils eine zur Aufnahme des zugehörigen Verbindungselementes ausgebildete, in radialer Richtung nach außen hin geöffnete Ausnehmung auf oder jeweils einen Greifer, wobei sich insbesondere vor dem Lösen der Gewindeverbindung die radiale Ausnehmung der ersten Aufnahmeeinrichtung in einem ersten definierten Winkelbereich in Umfangsrichtung befindet und sich die radiale Ausnehmung der zweiten Aufnahmeeinrichtung in einem zweiten Winkelbereich befindet, wobei sich der erste Winkelbereich und der zweite Winkelbereich zumindest teilweise überlappen. Dadurch kann auf besonders einfache und damit effiziente Art und Weise eine Gewindeverbindung in die Vorrichtung eingelegt werden, mittels derer zwei Schläuche miteinander verbunden sind, insbesondere erfindungsgemäß in radialer Richtung, so dass die Gewindeverbindung nicht aufgrund der Schläuche umständlich in axialer Richtung in die Vorrichtung eingebracht werden muss.

In einer weiteren vorteilhaften Ausgestaltung einer erfindungsgemäßen Vorrichtung ist die Vorrichtung in wenigstens zwei Betriebsmodi betreibbar, insbesondere in einem ersten Betriebsmodus zum Herstellen der Gewindeverbindung und in einem zweiten Betriebsmodus zum Lösen der Gewindeverbindung. Auf diese Art und Weise kann besonders einfach sichergestellt werden, dass zum Herstellen einer Gewindeverbindung die Aufnahmeeinrichtungen jeweils derart orientiert sind, dass nach dem Herstellen der Gewindeverbindung die Gewindeverbindung in radialer Richtung entnommen werden kann, wobei dazu nach dem Aktivieren des zugehörigen Betriebsmodus die Aufnahmeeinrichtungen der Vorrichtung vorzugsweise vor dem Verschrauben der beiden Verbindungselemente, besonders bevorzugt vor dem Einbringen der beiden Verbindungselemente, derart ausgerichtet werden, dass sie nach dem Verschrauben der beiden Verbindungselemente jeweils derart zueinander angeordnet sind, dass die Gewindeverbindung wie vorbeschrieben in radialer Richtung aus der Vorrichtung entnommen werden kann.

Zum Lösen der Gewindeverbindung sind die beiden Aufnahmeeinrichtungen vorzugsweise bereits vor dem Einbringen der Gewindeverbindung derart orientiert, dass die Gewindeverbindung in radialer Richtung in die Vorrichtung, wie vorbeschrieben, eingebracht werden kann. Die Erfindung wird im Anspruch 1 definiert und weitere Ausführungsformen finden sich in den abhängigen Ansprüchen.

Ein **erfindungsgemäßes Verfahren** zum Herstellen und/oder Lösen einer mit einem Fluid durchströmbaren, medizinischen Gewindeverbindung aus einem ersten Verbindungselement und einem zweiten Verbindungselement, vorzugsweise zum Herstellen und/oder Lösen einer Luer-Lock-Verbindung, mithilfe einer Vorrichtung mit einer ersten Aufnahmeeinrichtung und einer zweiten Aufnahmeeinrichtung, wobei die erste Aufnahmeeinrichtung und die zweite Aufnahmeeinrichtung um eine gemeinsame Verschraubungsachse relativ zueinander drehbar sind und entlang der Verschraubungsachse relativ zueinander verlagerbar sind, insbesondere mithilfe einer gemäß den Ansprüchen 1 bis 15 ausgebildeten Vorrichtung, weist die folgenden Schritte auf:
- Bereitstellen eines ersten Verbindungselementes und eines mit dem ersten Verbindungselement zu verbindenden, zweiten Verbindungselementes oder der zu lösenden Gewindeverbindung,
- Einbringen des ersten Verbindungselementes in die erste Aufnahmeeinrichtung der Vorrichtung,
- Einbringen des zweiten Verbindungselementes in die zweite Aufnahmeeinrichtung der Vorrichtung,
- Bewegen der ersten Aufnahmeeinrichtung derart relativ zur zweiten Aufnahmeeinrichtung, dass das erste Verbindungselement und das zweite Verbindungselement miteinander verschraubt werden oder die Gewindeverbindung gelöst wird.

Ein erfindungsgemäßes Verfahren ist dadurch gekennzeichnet, dass das erste Verbindungselement und/oder das zweite Verbindungselement oder die Gewindeverbindung bezogen auf die Verschraubungsachse in radialer Richtung in die zugehörige Aufnahmeeinrichtung eingebracht wird.

Vorzugsweise kann zum Herstellen der Gewindeverbindung die zweite Aufnahmeeinrichtung entlang der Verschraubungsachse verlagert werden, insbesondere mit der ersten Aufnahmeeinrichtung zusammengeschoben werden und um die Verschraubungsachse gedreht werden, insbesondere gleichzeitig unter einer von außen aufgebrachten Axialkraft.

Zum Lösen der Gewindeverbindung kann die zweite Aufnahmeeinrichtung vorzugsweise entlang der Verschraubungsachse von der ersten Aufnahmeeinrichtung weg verlagert werden und dabei insbesondere gleichzeitig um die Verschraubungsachse gedreht werden, vorzugsweise ebenfalls unter Axialkraftbelastung, jedoch mit entgegengesetzter Wirkrichtung. Dabei erfolgt vorzugsweise wenigstens das Bewegen der beiden Aufnahmeeinrichtungen relativ zueinander automatisiert, vorzugsweise gesteuert, insbesondere geregelt, wobei besonders bevorzugt das Relativbewegen der ersten Aufnahmeeinrichtung zur zweiten Aufnahmeeinrichtung drehmomentgesteuert oder drehmomentgeregelt und/oder kraftgesteuert oder kraftgeregelt und/oder drehwinkelgesteuert oder drehwinkelgeregelt und/oder weggesteuert und/oder weggeregelt erfolgt.

Vorzugsweise wird außerdem das erste Verbindungselement und/oder das zweite Verbindungselement automatisiert in die jeweils zugehörige Aufnahmeeinrichtung eingebracht. In einigen Fällen kann es auch vorteilhaft sein, wenn das Bereitstellen des ersten Verbindungselementes und/oder des zweiten Verbindungselementes ebenfalls automatisiert erfolgt.

Ist eine zur Durchführung eines erfindungsgemäßen Verfahrens verwendete Vorrichtung, insbesondere deren erste Aufnahmeeinrichtung und/oder deren zweite Aufnahmeeinrichtung, dabei nicht derart ausgebildet und angeordnet, dass die jeweils zugehörigen Verbindungselemente nach dem Einbringen in die zugehörige Aufnahmeeinrichtung derart zueinander ausgerichtet sind, dass die erste Achse des ersten Verbindungselementes und die zweite Achse des zweiten Verbindungselements zusammenfallen, insbesondere mit der Verschraubungsachse, werden vorzugsweise zunächst die erste Aufnahmeeinrichtung und die zweite Aufnahmeeinrichtung derart relativ zueinander bewegt, dass dies anschließend, d.h. nach dem Ausrichten der Fall ist. Dadurch können das erste Verbindungselement und das zweite Verbindungselement für die Herstellung der Gewindeverbindung oder das Lösen der Gewindeverbindung nahezu optimal angeordnet werden.

In einer vorteilhaften Ausgestaltung eines erfindungsgemäßen Verfahrens werden nach dem Herstellen der Gewindeverbindung das erste Verbindungselement und das zweite Verbindungselement der Gewindeverbindung jeweils in radialer Richtung aus der ersten Aufnahmeeinrichtung und der zweiten Aufnahmeeinrichtung entnommen, insbesondere in derselben radialen Richtung.

Nach dem Lösen der Gewindeverbindung wird einer vorteilhaften Ausgestaltung eines erfindungsgemäßen Verfahrens vorzugsweise wenigstens ein Verbindungselement in radialer Richtung entnommen, insbesondere werden vorzugsweise beide Verbindungselemente in radialer Richtung entnommen. In diesem Fall können die beiden Verbindungselemente jedoch problemlos auch in unterschiedlichen radialen Richtungen entnommen werden.

In einer weiteren vorteilhaften Ausgestaltung eines erfindungsgemäßen Verfahrens wird zum Herstellen der Gewindeverbindung, insbesondere vor dem Einbringen des ersten Verbindungselementes und/oder des zweiten Verbindungselementes ein erster Betriebsmodus aktiviert und/oder zum Lösen der Gewindeverbindung, insbesondere vor dem Einbringen der Gewindeverbindung, ein zweiter Betriebsmodus. Dadurch kann auf besonders einfache Art und Weise die Vorrichtung jeweils in eine optimale Position zum Einbringen der beiden Verbindungselemente gebracht werden.

Weitere mögliche bevorzugte Ausgestaltungen des erfindungsgemäßen Verfahrens lassen sich aus der Beschreibung des erfindungsgemäßen Systems und von dessen bevorzugten Ausgestaltungen ableiten.

Die mit Bezug auf eine erfindungsgemäße Vorrichtung zum Herstellen und/oder Lösen einer mit einem Fluid durchströmbaren, medizinischen Gewindeverbindung vorgestellten, vorteilhaften Ausgestaltungen und deren Vorteile gelten entsprechend auch für ein erfindungsgemäßes Verfahren einer solchen Gewindeverbindung.

Die vorliegende Erfindung bezieht sich auch auf einen Kanülierautomaten, der eine erfindungsgemäße Vorrichtung (100, 200, 200`, 300) gemäß mindestens einer der hier beschriebenen möglichen Ausgestaltungen zum Herstellen und/oder Lösen einer mit einem Fluid durchströmbaren, medizinischen Gewindeverbindung aufweist, und der insbesondere zur Durchführung eines erfindungsgemäßen Verfahrens eingerichtet ist. Auf diese Weise ist der Kanülierautomat insbesondere dazu eingerichtet, medizinische Fluidverbindungen, insbesondere Luer-Lock-Verbindungen, automatisiert und autonom herzustellen, insbesondere nachdem zuvor bereits die automatische Punktion des Blutgefäßes des Patienten durch den Kanülierautomat durchgeführt wurde und insbesondere nachdem die punktierende Kanüle an der Haut des Patienten durch den Kanülierautomat mittels Klebeband oder einer anderen Fixiereinrichtung automatisch fixiert wurde.

Ein Kanülierautomat ist eine Vorrichtung, die mindestens einen Prozessschritt der Kanülierung eines Blutgefäßes eines Patienten, oder einige oder alle dafür vorgesehenen Prozessschritte automatisch, das heißt zumindest zeitweise oder ununterbrochen, ohne Eingriff eines menschlichen Bedieners, z.B. medizinischen Personals, durchführt. Dieser Prozesschritt ist insbesondere die automatische Punktion des Blutgefäßes, insbesondere einer arteriovenösen Fistel; vorzugsweise werden für eine Blutentnahme aus dem Blutgefäß eine erste Punktion und Kanülierung und für die Blutrückführung in das Blutgefäß eine zweite Punktion und Kanülierung automatisch durchgeführt, insbesondere bei der Hämodialyse; die Programmparameter der automatisierten Kanülierung können hierbei in Abhängigkeit von der angemeldeten Patientenkennung, also patientenindividuell, gewählt sein, indem durch die Programmparameter eine patientenabhängige Bewegungssteuerung für einen optional beim Kanülierautomaten vorgesehenen robotergesteuerten Werkzeugarm definiert wird, mittels der zum Beispiel ein medizinisches Zubehör wie etwa eine Injektionsnadel vom Werkzeugarm gegriffen und am Körperteil positioniert wird, wobei die Injektionsnadel zuvor patientenspezifisch ausgewählt und vorbereitet wurde; zwei Kanülierautomaten können für die Punktion von Blutgefäßen an unterschiedlichen Körperteilen eingerichtet sein, indem z.B. ein erster Kanülierautomat für die Kanülierung an einem Arm eingerichtet sein kann und ein zweiter Kanülierautomat für die Kanülierung an einem Bein eingerichtet sein kann; die Auswahl des geeigneten Kanülierautomaten kann patientenspezifisch und/oder behandlungsspezifisch erfolgen.

Weitere bevorzugte Ausgestaltungen des erfindungsgemäßen Systems und des erfindungsgemäßen Verfahrens ergeben sich aus der nachfolgenden Beschreibung der Ausführungsbeispiele in Zusammenhang mit den Figuren und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt.

Es zeigen:
- Fig. 1a bis 1c:: schematisch ein erstes Ausführungsbeispiel eines aus dem Stand der Technik bekannten Luer-Lock-Verbindungselementes mit einem männlichen Luer-Lock-Verbindungsabschnitt in verschiedenen Ansichten,
- Fig. 2a bis 2c:: schematisch ein erstes Ausführungsbeispiel eines aus dem Stand der Technik bekannten Luer-Lock-Verbindungselementes mit einem weiblichen Luer-Lock-Verbindungsabschnitt in verschiedenen Ansichten,
- Fig. 3a bis 3c:: schematisch ein zweites Ausführungsbeispiel eines aus dem Stand der Technik bekannten Luer-Lock-Verbindungselementes mit einem weiblichen Luer-Lock-Verbindungsabschnitt in verschiedenen Ansichten,
- Fig. 4:: schematisch ein erstes Ausführungsbeispiel einer erfindungsgemäßen Vorrichtung zum Herstellen einer Luer-Lock-Gewindeverbindung in Draufsicht,
- Fig. 5:: die erfindungsgemäße Vorrichtung aus Fig. 4 in Prinzipdarstellung von vorne,
- Fig. 6a:: das Luer-Lock-Verbindungselement aus Fig. 2a vor dem Einbringen in die zweite Aufnahmeeinrichtung der erfindungsgemäßen Vorrichtung aus den Fig. 4 und 5,
- Fig. 6b:: die erfindungsgemäße Vorrichtung aus Fig. 4 und 5 in Prinzipdarstellung in Ansicht von der Seite vor dem Einbringen des Luer-Lock-Verbindungselements aus Fig. 6a in die zweite Aufnahmeeinrichtung,
- Fig. 6c:: die erfindungsgemäße Vorrichtung aus Fig. 4 und 5 in Prinzipdarstellung in Ansicht von vorne nach dem Einbringen des Luer-Lock-Verbindungselements aus Fig. 6a in die zweite Aufnahmeeinrichtung, jedoch vor dem Herstellen der Luer-Lock-Gewindeverbindung,
- Fig. 7:: schematisch ein zweites Ausführungsbeispiel einer erfindungsgemäßen Vorrichtung zum Herstellen einer Luer-Lock-Gewindeverbindung in Seitenansicht nach dem Einbringen des Luer-Lock-Verbindungselements aus Fig. 2a in die zweite Aufnahmeeinrichtung, jedoch vor dem Herstellen der Luer-Lock-Gewindeverbindung,
- Fig. 8a:: schematisch ein drittes Ausführungsbeispiel einer erfindungsgemäßen Vorrichtung zum Herstellen einer Luer-Lock-Gewindeverbindung in Seitenansicht vor dem Einbringen eines Luer-Lock-Verbindungselements in die zweite Aufnahmeeinrichtung in einem ersten Zustand,
- Fig. 8b:: die Vorrichtung aus Fig. 8a in einem zweiten Zustand und
- Fig. 9:: schematisch ein viertes Ausführungsbeispiel einer erfindungsgemäßen Vorrichtung zum Herstellen einer Luer-Lock-Gewindeverbindung

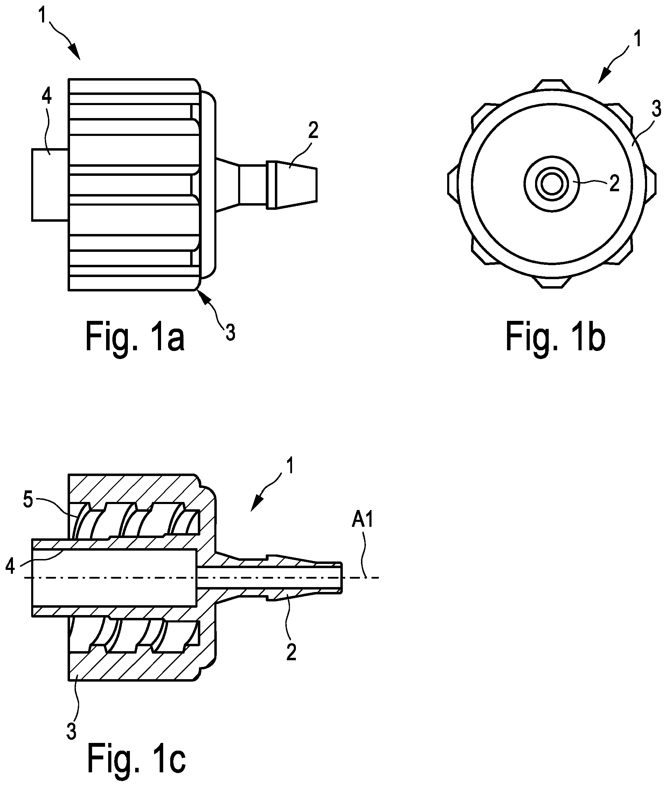

Die Figuren 1a bis 1c zeigen jeweils schematisch eine Ansicht eines ersten Ausführungsbeispiels eines aus dem Stand der Technik bekannten, Luer-Lock-Verbindungselements 1 mit einem männlichen Luer-Lock-Verbindungsabschnitt 5, wobei in Figur 1a ein Luer-Lock-Verbindungselement in Seitenansicht, in Figur 1b die Ansicht von hinten und in Figur 1c im Schnitt dargestellt ist.

Das Luer-Lock-Verbindungselement 1 weist dabei einen Schlauchanschlussabschnitt 2 sowie einen hülsenförmigen, männlichen Luer-Lock-Verbindungsabschnitt 3 mit einem Innengewinde 5 auf, welches konzentrisch zu einem Rohrabschnitt 4 um eine erste Achse A1 angeordnet ist. Der Rohrabschnitt 4 weist dabei, wie bei Luer-Verbindungselementen üblich, einen Außenkonus mit einer 6%-Steigung auf und kann, ebenso wie der Schlauchanschlussabschnitt 2, von einem fluiden Medium durchströmt werden. Der hülsenförmige, männliche Luer-Lock-Verbindungsabschnitt 3 weist an seiner Außenseite mehrere, hier nicht näher bezeichnete, in Umfangsrichtung verteilt angeordnete Rippen auf.

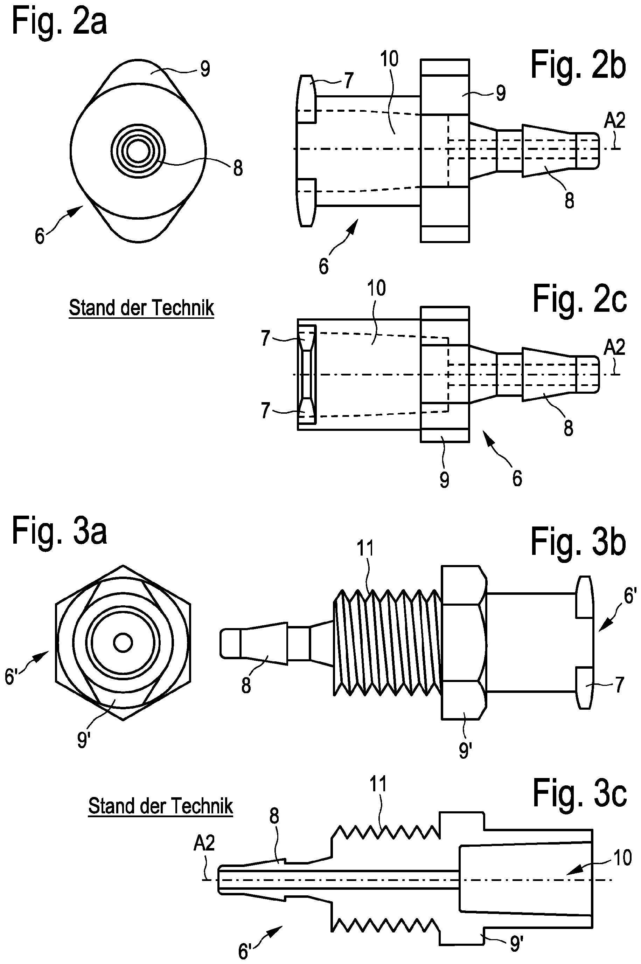

Die Figuren 2a bis 2c zeigen jeweils schematisch eine Ansicht eines ersten Ausführungsbeispiels eines aus dem Stand der Technik bekannten Luer-Lock-Verbindungselementes 6 mit einem weiblichen Luer-Lock-Verbindungsabschnitt 10, wobei Figur 2a eine Ansicht von hinten, Figur 2b eine Draufsicht und Figur 2c eine Seitenansicht zeigt.

Das Luer-Lock-Verbindungselement 6 weist dabei einen, als Innenkegel ausgebildeten, weiblichen Luer-Lock-Verbindungsabschnitt 10 auf, welcher ebenfalls eine 6%-Steigung aufweist. Das Luer-Lock-Verbindungselement 6 weist ebenfalls einen Schlauchanschlussabschnitt 8 auf. Der Innenkegel 10 sowie der Schlauchanschlussabschnitt 8 sind ebenfalls mit einem fluiden Medium durchströmbar.

Zur Verbindung des weiblichen Luer-Lock-Verbindungselements 6 mit einem männlichen Luer-Lock-Verbindungselement 1, wie es in den Figuren 1a bis 1c beispielhalber dargestellt ist, weist das in den Figuren 2a bis 2c dargestellte Luer-Lock-Verbindungselement 6 einen sich in radialer Richtung nach außen erstreckenden Flanschabschnitt mit einem Außengewinde 7 auf, wobei das Außengewinde 7 sowie der Innenkegel 10 konzentrisch zu einer zweiten Achse A2 des Verbindungselementes 6 angeordnet sind.

Für eine besonders einfache Handhabung des weiblichen Luer-Lock-Verbindungselementes 6 weist dieses in diesem Fall in etwa in der Mitte, bezogen auf seine Längserstreckung; in Richtung der zweiten Achse A2 eine Handhabungs-Kontur 9 in Form von zwei Flügeln auf welche jeweils in etwa die Geometrie einer halben Raute mit abgerundeten Ecken aufweisen. Weibliche Luer-Lock-Verbindungselemente 6 mit einer derartigen Handhabungs-Kontur 9 sind aus dem Stand der Technik grundsätzlich bekannt, so dass auf weitere Ausführungen an dieser Stelle verzichtet wird.

Die Figuren 3a bis 3c zeigen jeweils schematisch, ebenfalls in verschiedenen Ansichten, ein zweites Ausführungsbeispiel eines aus dem Stand der Technik bekannten Luer-Lock-Verbindungselementes 6', welches ebenfalls einen weiblichen Luer-Lock-Verbindungsabschnitt 10 aufweist.

Dieses Luer-Lock-Verbindungselement 6` unterscheidet sich dabei in der Handhabungs-Kontur 9' sowie einem zusätzlichen Anschlussgewinde 11, welches angrenzend an den Schlauchanschlussabschnitt 8 und die Handhabungs-Kontur 9' angeordnet ist, insbesondere zwischen dem Schlauchanschlussabschnitt 8 und der Handhabungs-Kontur 9'.

Ansonsten ist das in den Figuren 3a bis 3c dargestellte, weibliche Luer-Lock-Verbindungselement ähnlich wie das anhand der Figuren 2a bis 2c beschriebene Luer-Lock-Verbindungselement 6 ausgebildet. Insbesondere ist der weibliche Anschlussabschnitt 10 mit dem konusförmigen Innenkegel und dem Außengewinde 7 identisch zu dem Luer-Lock-Verbindungselement 6 aus den Figur 2a bis 2c ausgebildet, wie es auch die entsprechenden Normen für Luer-Lock-Verbindungselemente vorsehen, welche lediglich verschiedene Größen zulassen, die einzelnen Abmessungen jedoch in einem bestimmten Verhältnis zueinander vorzusehen sind.

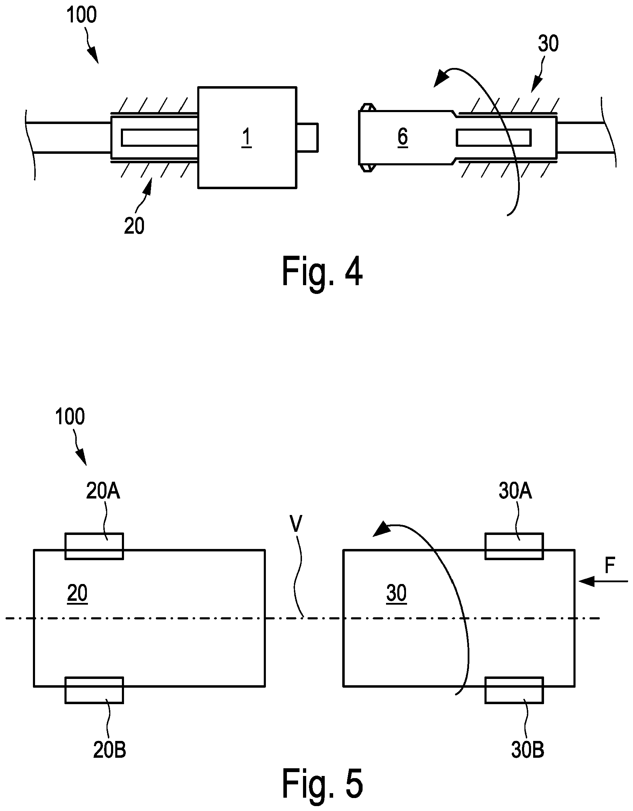

Figur 4 zeigt schematisch ein erstes Ausführungsbeispiel einer erfindungsgemäßen Vorrichtung 100 zum Herstellen einer Luer-Lock-Gewindeverbindung in Draufsicht, wobei die erfindungsgemäße Vorrichtung 100 eine erste Aufnahmeeinrichtung 20 sowie eine zweite Aufnahmeeinrichtung 30 aufweist.

Die erste Aufnahmeeinrichtung 20 ist in diesem Fall als feste Einspannung und zur Aufnahme eines ersten Verbindungselementes 1 ausgebildet, welches beispielhalber durch das zuvor beschriebene, aus dem Stand der Technik bekannte, männliche Luer-Lock-Verbindungselement 1 mit einem männlichen Luer-Lock-Verbindungsabschnitt dargestellt ist.

Die feste Einspannung der ersten Aufnahmevorrichtung 20 wird durch eine obere Lagerschale 20A sowie durch eine untere Lagerschale 20B bewirkt, mittels derer die erste Aufnahmeeinrichtung ortsfest innerhalb der erfindungsgemäßen Vorrichtung 100 angeordnet ist, siehe Figur 5.

Die zweite Aufnahmeeinrichtung 30 ist zur Aufnahme eines zweiten Verbindungselementes ausgebildet ist, was hier durch das zweite, zuvor beschriebene, aus dem Stand der Technik bekannte Luer-Lock-Verbindungselement 6 mit einem weiblichen Luer-Lock-Verbindungsabschnitt dargestellt ist.

Die zweite Aufnahmeeinrichtung 30 ist dabei durch eine Gewindespindel gebildet, welche durch die Pfeilrichtung symbolisiert ist und welche um eine hier nicht näher bezeichnete Verschraubungsachse drehbar und entlang der Verschraubungsachse axial verlagerbar ist.

Dabei weist die zweite Aufnahmeeinrichtung 30 ein hier nicht dargestelltes, nicht selbsthemmend ausgebildetes Außengewinde auf, welches mit einem entsprechend korrespondierend ausgebildeten, ebenfalls nicht dargestellten Innengewinde einer oberen Lagerschale 30A und einer unteren Lagerschale 30B im Eingriff ist und eine axiale Führung der Gewindespindel 30 entlang der Verschraubungsachse V ermöglicht.

Für eine ausreichend genaue Führung der Gewindespindel 30 bzw. für eine ausreichend stabile Lagerung der ersten Aufnahmeeinrichtung 20 kann es in einigen Fällen erforderlich sein, jeweils mehr als ein Lager vorzusehen mit entsprechend ausgebildeten Lagerschalen 20A, 20B, 30A und 30B.

Aufgrund der nicht selbsthemmenden Ausgestaltung der Gewindespindel 30 kann durch Aufbringen einer Axialkraft F auf die Gewindespindel eine Drehbewegung um die Verschraubungsachse V erzeugt werden, wobei die Gewindespindel 30 infolge der Drehbewegung in axialer Richtung entlang der Verschraubungsachse V verlagert wird.

Zum Verschrauben des ersten Verbindungselementes 1 und des zweiten Verbindungselementes 6 kann die zweite Aufnahmeeinrichtung 30 in axialer Richtung entlang der Verschraubungsachse V bewegt werden, insbesondere durch Aufbringen einer ausreichenden Axialkraft F.

Die Axialkraft F kann dabei vorzugsweise mittels einer entsprechend ausgebildeten, hier nicht dargestellten Aktuatorvorrichtung erzeugt werden, beispielsweise mit einem Antriebsmotor und einem Zahnstangengetriebe, wobei vorzugsweise ein Ritzel des Zahnstangengetriebe drehfest mit dem Antriebsmotor verbunden ist und mittels des Antriebsmotors in Drehung versetzt werden kann, wodurch eine mit dem Ritzel kämmende Zahnstange axial verlagert werden kann, was zum Aufbringen einer Axialkraft auf die Gewindespindel 30 genutzt werden kann.

Ist die Gewindespindel 30 hingegen selbsthemmend ausgebildet bzw. der Spindeltrieb aus Gewindespindel 30 und den zugehörigen Lagerschalen 30A und 30B, welche die Gewindespindel führen, ist das Erzeugen einer Axialbewegung der Gewindespindel 30 durch Aufbringen einer Axialkraft F nicht möglich, da in diesem Fall auf die Gewindespindel 30 aufgebrachte Axialkräfte über die Lagerschalen 30A und 30B abgestützt werden. In diesem Fall kann eine Drehbewegung der Gewindespindel nur durch Drehen der Gewindespindel 30 bewirkt werden, wodurch in diesem Fall die zum Verschrauben der beiden Verbindungselemente 1 und 6 erforderliche Axialbewegung erzeugt werden kann.

Die erste Aufnahmeeinrichtung 20 und die zweite Aufnahmeeinrichtung 30 sind bei dieser erfindungsgemäßen Vorrichtung 100 derart zueinander angeordnet und ausgebildet, dass die jeweiligen, zugehörigen Luer-Lock-Verbindungselemente 1 und 6 jeweils derart aufgenommen werden können, dass eine erste Achse A1 des ersten Verbindungselementes 1 und eine zweite Achse A2 des zweiten Verbindungselementes 6 unmittelbar nach dem Einbringen in die Vorrichtung 100 miteinander fluchten und insbesondere mit der Verschraubungsachse V zusammenfallen, so dass das erste Verbindungselement 1 und das zweite Verbindungselement 6 unmittelbar im Anschluss an das Einbringen miteinander verschraubt werden können.

Die aufgebrachte Axialkraft F bewirkt infolge der nicht selbsthemmend ausgebildeten Gewindespindel 30 eine Drehbewegung der Gewindespindel 30 um die Verschraubungsachse V, wodurch gleichzeitig die zweite Aufnahmeeinrichtung 30, welche durch eine radiale Ausnehmung 40 in der Gewindespindel 30 gebildet ist, um die Verschraubungsachse V gedreht wird. Durch die Drehbewegung der Gewindespindel 30 wiederum wird diese in axialer Richtung hin zu der ersten Aufnahmeeinrichtung 20 bewegt.

Dadurch kann, sofern sich die Drehbewegung um die Verschraubungsachse V über einen ausreichend großen Drehwinkelbereich erstreckt bzw. über einen ausreichend großen Drehwinkelbereich ausgeführt wird, ein in der zweiten Aufnahmeeinrichtung 30 aufgenommenes zweites Verbindungselement 6 mit einem in der ersten Aufnahmeeinrichtung 20 aufgenommen ersten Verbindungselement 1 auf eine besonders einfache Art und Weise, insbesondere automatisiert, verschraubt werden und eine Gewindeverbindung hergestellt werden.

Entsprechend kann durch Aufbringen einer entgegengesetzt gerichteten Axialkraft eine Drehbewegung der zweiten Aufnahmeeinrichtung 30 in die entgegengesetzte Richtung bewirkt werden bei gleichzeitiger axialer Verlagerung der Gewindespindel 30 weg von der ersten Aufnahmeeinrichtung 20. Dadurch kann eine Gewindeverbindung gelöst werden

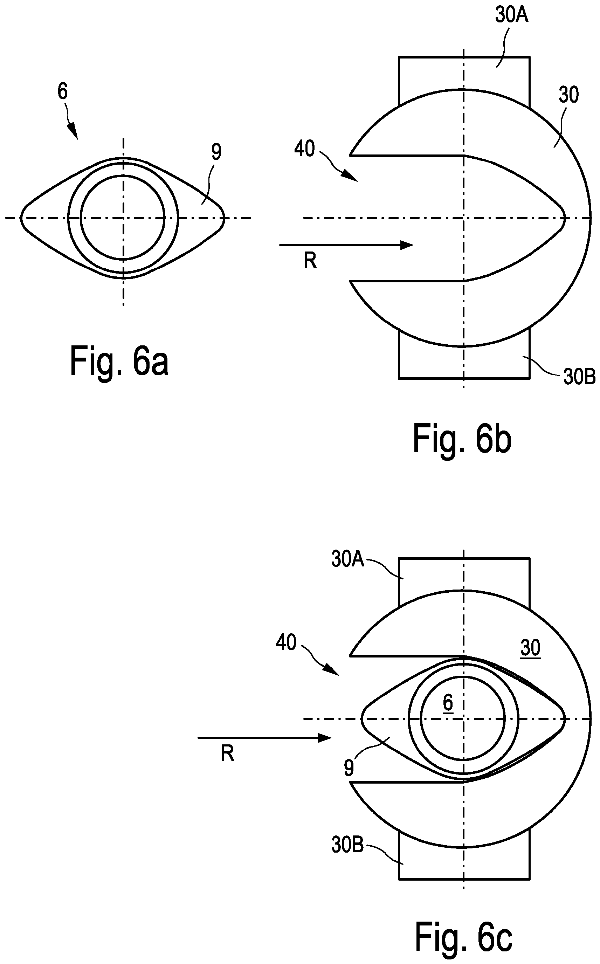

Figur 6a zeigt das Verbindungselement 6 aus Figur 2a in Einzelteildarstellung vor dem Einbringen die zweite. Aufnahmeeinrichtung 30 der erfindungsgemäßen Vorrichtung 100 aus den Figuren 4 und 5.

Figur 6b zeigt die erfindungsgemäße Vorrichtung 100 aus den Figuren 4 und 5 in Prinzipdarstellung in Ansicht von der Seite vor dem Einbringen des Verbindungselementes 6 aus Figur 6a in die zweite Aufnahmeeinrichtung 30.

In dieser Darstellung ist gut zu erkennen, dass die zweite Aufnahmeeinrichtung 30 eine zur Aufnahme des zweiten Verbindungselements 6 ausgebildete, in radialer Richtung R nach außen hin geöffnete Ausnehmung 40 aufweist welche insbesondere an die Handhabungs-Kontur 9 des zweiten Verbindungselementes 6 zumindest teilweise angepasst ist. Dadurch kann auf besonders einfache Art und Weise nicht nur das zweite Verbindungselement 6 von der zweiten Aufnahmeeinrichtung 30 aufgenommen werden, sondern außerdem noch formschlüssig gegen ein Verdrehen um die hier nicht näher bezeichnete Verschraubungsachse V, welche sich in dieser Darstellung senkrecht zur Zeichenebene erstreckt, gesichert werden. Dies ist anhand von Figur 6c, welche die erfindungsgemäße Vorrichtung 100 aus den Figuren 4 und 5 sowie 6b nach dem Einbringen des Luer-Lock-Verbindungselements 6 in die zweite Aufnahmeeinrichtung 30 jedoch vor dem Herstellen der Luer-Lock-Gewindeverbindung zeigt, gut zu erkennen.

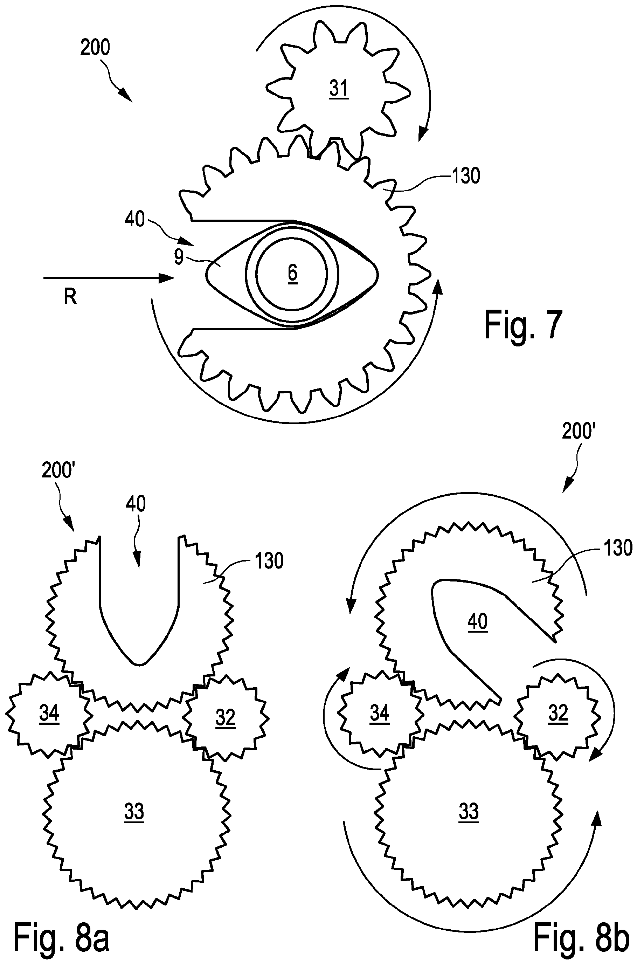

Figur 7 zeigt schematisch ein zweites Ausführungsbeispiel einer erfindungsgemäßen Vorrichtung 200 zum Herstellen und Lösen einer Luer-Lock-Gewindeverbindung in Seitenansicht nach dem Einbringen des Luer-Lock-Verbindungselementes 6 aus Figur 2a in die zweite Aufnahmeeinrichtung 130, jedoch vor dem Herstellen der Lock-Luer-Gewindeverbindung.

In diesem Fall ist die zweite Aufnahmeeinrichtung 130 durch ein erstes Getriebeelement 130 in Form eines geradverzahnten Stirnrads 130 gebildet, welches mithilfe eines zweiten Getriebeelements 31, das ebenfalls als geradverzahntes Stirnrad 31 ausgebildet ist, und das mit einem ersten Antriebsmotor drehverbunden ist und mittels diesem in eine Drehbewegung versetzt werden kann, kämmt.

Zur Erzeugung der zum Herstellen der Gewindeverbindung neben der Drehbewegung außerdem erforderlichen relativen Axialbewegung zwischen der hier nicht dargestellten ersten Aufnahmeeinrichtung und der zweiten Aufnahmeeinrichtung 130 weist die erfindungsgemäße Vorrichtung 200 hier nicht dargestelltes ein Federspannsystem bzw. eine Federspannvorrichtung auf, welche dazu ausgebildet ist, die Verbindungseinrichtung 130 in axialer Richtung derart relativ zu der ersten Aufnahmeeinrichtung zu verlagern, dass das zweite Verbindungselement 6 mit dem zugehörigen, korrespondierend ausgebildeten anderen Verbindungselement 1, welches von der ersten Aufnahmeeinrichtung aufgenommen ist, zu einer Gewindeverbindung verschraubt werden kann.

Eine Drehbewegung des zweiten Stirnrads 31 im Uhrzeigersinn führt dabei zu einer Drehbewegung des ersten Stirnrads 130 und damit der ersten Aufnahmeeinrichtung 130, welche durch die radiale Ausnehmung 40 im ersten Getriebeelement 31 gebildet ist, entgegen dem Uhrzeigersinn.

Wie bei der zuvor beschriebenen erfindungsgemäßen Vorrichtung 100 ist die Ausnehmung 40 auch in diesem Fall korrespondierend zur Handhabungs-Kontur 9 des zweiten Verbindungselementes 6 ausgebildet und insbesondere derart ausgebildet, dass das zweite Verbindungselement 6 verdrehgesichert in der zweiten Aufnahmeeinrichtung 130 angeordnet werden kann.

Ist der zum Herstellen der Gewindeverbindung erforderliche Drehwinkel dabei größer als der in Umfangsrichtung des ersten Getriebeelements 130 mit einer Außenverzahnung ausgebildete Bereich, d.h. größer als 360° minus des Drehwinkelbereichs der Ausnehmung 40, kann die Gewindeverbindung mittels der in Figur 7 dargestellten erfindungsgemäßen Vorrichtung 200 nicht ordnungsgemäß verschraubt werden, da im Bereich der Ausnehmung 40 kein Antrieb mittels des zweiten Getriebeelementes 31 möglich ist. Insbesondere wenn der Winkelbereich im Bereich der Öffnung 40 dabei derart groß ist, dass das zweite Getriebeelement 31 zumindest teilweise außer Eingriff gerät.

Für diesen Anwendungsfall eignet sich insbesondere die in den Figuren 8a und 8b dargestellte, erfindungsgemäße Vorrichtung 200', welche neben dem ersten Getriebeelement 130, welches die zweite Aufnahmeeinrichtung 130 bildet und wie bei den beiden zuvor beschriebenen erfindungsgemäßen Vorrichtung 100 und 200 jeweils eine Ausnehmung 40 in radialer Richtung aufweist, ein zweites Getriebeelement 32 und noch ein drittes Getriebeelement 34 aufweist, welches ebenfalls mit dem ersten Getriebeelement 130 drehverbunden ist, und insbesondere ebenfalls mit dem ersten Getriebeelement 130 im Eingriff ist bzw. kämmt.

In diesem Fall sind das zweite Getriebeelement 32 und das dritte Getriebeelement 34 über ein, weiteres, viertes Getriebeelement 33 miteinander wirkverbunden, so dass eine von einem ersten Antriebsmotor auf das zweite Getriebeelement 32 aufgebrachte Antriebsleistung zum einen über das zweite Getriebeelement 32 an das erste Getriebeelement 31 übertragen werden kann, zum anderen über das vierte Getriebeelement 33 und das dritte Getriebeelement 34 an das erste Getriebeelement 31.

Insbesondere, wenn sich die Ausnehmung 40 im Bereich des zweiten Getriebeelements 32 befindet, wie zum besseren Verständnis in Figur 8b dargestellt, kann über das vierte Getriebeelement 33 und das dritte Getriebeelement 34 eine mittels eines Antriebsmotors erzeugte Antriebsleistung an das erste Getriebeelement 130 übertragen werden. Dadurch kann der nicht verzahnte Winkelbereich im Bereich der Ausnehmung 40 überbrückt werden.

Ist, wie bei dem anhand der Figuren 8a und 8b erläuterten Ausführungsbeispiel einer erfindungsgemäßen Vorrichtung 200', nur ein Antriebsmotor zum Antrieb des zweiten Getriebeelementes 32 vorgesehen, ist es aufgrund der erforderlichen Drehrichtungsumkehr nötig, das dritte Getriebeelement 34 über ein viertes Getriebeelement 33 mit dem zweiten Getriebeelement 32 zu koppeln, was insbesondere anhand der Drehrichtungspfeile in Figur 8b erkennbar ist.

Dreht sich nämlich das zweite Getriebeelement 32 im Uhrzeigersinn, ergibt sich eine Drehrichtung des vierten Getriebeelements 33 gegen den Uhrzeigersinn wodurch sich das dritte Getriebeelement 34 wiederum im Uhrzeigersinn dreht, genau wie das zweite Getriebeelement 32, so dass ein Blockieren des Getriebes vermieden werden kann, wenn sowohl das zweite Getriebeelement 32 als auch das dritte Getriebeelement 34 mit dem ersten Getriebeelement 130 im Eingriff sind.

Alternativ ist aber auch möglich, statt des vierten Getriebeelement 33 einen zweiten Antriebsmotor vorzusehen und diesen zum Antrieb des dritten Getriebeelements 34 zu nutzen, wobei in diesem Fall die Antriebsleistung mit der gleichen Drehrichtung aufgebracht werden muss die beim zweiten Getriebeelement 33, insbesondere drehzahlsynchronisiert auf das erste Getriebeelement 130.

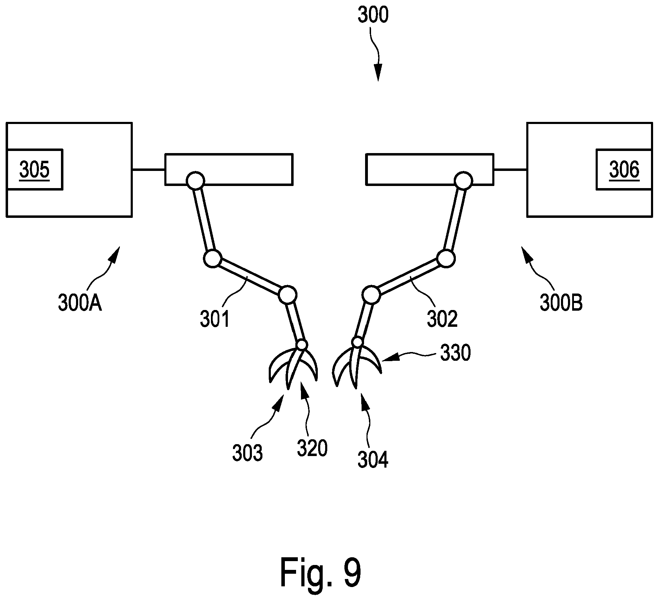

Figur 9 zeigt schematisch ein viertes Ausführungsbeispiel einer erfindungsgemäßen Vorrichtung 300 zum Herstellen und/oder Lösen einer Luer-Lock-Gewindeverbindung, wobei diese erfindungsgemäße Vorrichtung 300 eine erste Greifervorrichtung 300A und eine zweite Greifervorrichtung 300B aufweist. Die erste Greifervorrichtung 300A weist dabei einen Greiferarm 301 mit einer am freien Ende des Greiferarmes 301 befestigten, ersten Aufnahmeeinrichtung 320 auf, welche durch ein Greifer 303 gebildet ist, wobei zur Steuerung der ersten Greifervorrichtung 300A die Steuerungseinrichtung 300B vorgesehen ist.

Die zweite Greifervorrichtung 300B ist analog zur ersten Greifervorrichtung 300A ausgebildet und weist ebenfalls einen Greiferarm 302 auf, an dessen freien Ende eine zweite Aufnahmeeinrichtung 330 befestigt ist, welche durch einen Greifer 304 gebildet ist. Zur Steuerung der zweiten Greifervorrichtung 300B ist entsprechend die Steuerungseinrichtung 306 vorgesehen.

Selbstverständlich ist eine Vielzahl an Abwandlungen, insbesondere konstruktiver Art, möglich, ohne den Inhalt der Patentansprüche zu verlassen

### Bezugszeichenliste:

- 1: Luer-Lock-Verbindungselement mit männlichem Luer-Lock-Verbindungsabschnitt
- 2: Schlauchanschlussabschnitt
- 3: hülsenförmiger, männlicher Luer-Lock-Verbindungsabschnitt mit Innengewinde
- 4: Rohrabschnitt mit Außenkonus
- 5: Innengewinde
- 6, 6': Luer-Lock-Verbindungselement mit weiblichen Luer-Lock-Verbindungsabschnitt
- 7: Außengewinde
- 8: Schlauchanschlussabschnitt
- 9, 9': Handhabungs-K ontur
- 10: weiblicher Luer-Lock-Verbindungsabschnitt mit Innenkonus
- 11: Anschlussgewinde
- 20: erste Aufnahmeeinrichtung
- 20A: obere Lagerschale der ersten Aufnahmeeinrichtung
- 20B: untere Lagerschale der ersten Aufnahmeeinrichtung
- 30: zweite Aufnahmeeinrichtung, Gewindespindel
- 30A: obere Lagerschale der zweiten Aufnahmeeinrichtung
- 30B: untere Lagerschale der zweiten Aufnahmeeinrichtung
- 31: zweites Getriebeelement
- 32: zweites Getriebeelement
- 33: viertes Getriebeelement
- 34: drittes Getriebeelement
- 40: Ausnehmung
- 100, 200, 200', 300: erfindungsgemäße Vorrichtung
- 130: zweite Aufnahmeeinrichtung; erstes Getriebeelement
- 300A: erste Greifervorrichtung
- 300B: zweite Greifervorrichtung
- 301: Greiferarm der ersten Greifervorrichtung
- 302: Greiferarm der zweiten Greifervorrichtung
- 303: Greifer der ersten Aufnahmeeinrichtung
- 304: Greifer der zweiten Aufnahmeeinrichtung
- 305: Steuerungseinrichtung zur Steuerung der ersten Greifervorrich-tung
- 306: Steuerungseinrichtung zur Steuerung der zweiten Greifervor-richtung
- 320: erste Aufnahmeeinrichtung
- 330: zweite Aufnahmeeinrichtung
- A1: erste Achse
- A2: zweite Achse
- F: Axialkraft
- R: radiale Richtung
- V: Verschraubungsachse

## Patentansprüche

1. Vorrichtung (100, 200, 200', 300) zum Herstellen und/oder Lösen einer mit einem Fluid durchströmbaren, medizinischen Gewindeverbindung aus einem ersten Verbindungselement (1) und einem zweiten Verbindungselement (6, 6'), insbesondere zum Herstellen und/oder Lösen einer Luer-Lock-Verbindung,
wobei die Vorrichtung (100, 200, 200', 300) eine erste Aufnahmeeinrichtung (20, 320) zur Aufnahme des ersten Verbindungselements (1) und eine zweite Aufnahmeeinrichtung (30, 130, 330) zur Aufnahme des zweiten Verbindungselements (6, 6') aufweist,
wobei die erste Aufnahmeeinrichtung (20, 320) und die zweite Aufnahmeeinrichtung (30, 130, 330) um eine gemeinsame Verschraubungsachse (V) relativ zueinander drehbar sind und entlang der Verschraubungsachse (V) relativ zueinander verlagerbar sind,
und wobei die Vorrichtung (100, 200, 200', 300) dazu eingerichtet ist, nach dem Einbringen des ersten Verbindungselementes (1) in die erste Aufnahmeeinrichtung (20, 320) und dem Einbringen des zweiten Verbindungselementes (6, 6') in die zweite Aufnahmeeinrichtung (30, 130, 330), die erste Aufnahmeeinrichtung (20, 320) und die zweite Aufnahmeeinrichtung (30, 130, 330) derart relativ zueinander zu bewegen, dass das erste Verbindungselement (1) und das zweite Verbindungselement (6, 6') miteinander verschraubt werden oder die Gewindeverbindung gelöst wird,
**dadurch gekennzeichnet, dass**
die erste Aufnahmeeinrichtung (20) und/oder die zweite Aufnahmeeinrichtung (30, 13) eine zur Aufnahme des zugehörigen Verbindungselementes (1; 6, 6') ausgebildete, bezogen auf die Verschraubungsachse (V) in radialer Richtung nach außen hin geöffnete Ausnehmung (40) aufweist, oder
die erste Aufnahmeeinrichtung (320) und/oder die zweite Aufnahmeeinrichtung (330) einen Greifer (303, 304) aufweist oder als Greifer (303, 304) ausgebildet ist,
so dass in die erste Aufnahmeeinrichtung (20, 320) und/oder in die zweite Aufnahmeeinrichtung (30, 130, 330) das zugehörige Verbindungselement (1; 6, 6') bezogen auf die Verschraubungsachse (V) in radialer Richtung (R) einbringbar ist.

2. Vorrichtung (100, 200, 200`, 300) nach Anspruch 1, **dadurch gekennzeichnet, dass** die Vorrichtung (100, 200, 200', 300) eine Aktuator-Einrichtung zur Erzeugung der Relativbewegung zwischen der ersten Aufnahmeeinrichtung (20, 320) und der zweiten Aufnahmeeinrichtung (30, 130, 330) zum zumindest teilweise automatisierten Herstellen und/oder Lösen der Gewindeverbindung aufweist.

3. Vorrichtung (100, 200, 200`, 300) nach wenigstens einem der vorgenannten Ansprüche, **dadurch gekennzeichnet, dass** eine der beiden Aufnahmeeinrichtungen (20, 320; 30, 130, 330), insbesondere die erste Aufnahmeeinrichtung (20, 320), zur Aufnahme eines ersten Verbindungselements (1) mit einem ersten Verbindungsabschnitt (3) mit einem konzentrisch zu einer ersten Achse (A1) angeordneten, hülsenförmigen Innengewinde (5) ausgebildet ist, insbesondere zur Aufnahme eines ersten Verbindungselementes (1) mit einem männlichen Luer-Lock-Verbindungsabschnitt (3), und die andere Aufnahmeeinrichtung (20, 320; 30, 130, 330), insbesondere die zweite Aufnahmeeinrichtung (30, 130, 330), zur Aufnahme eines zweiten Verbindungselements (6, 6') mit einem zweiten Verbindungsabschnitt (10) mit einem konzentrisch zu einer zweiten Achse (A2) angeordneten Außengewinde (7), insbesondere zur Aufnahme eines zweiten Verbindungselementes (6, 6') mit einem weiblichen Luer-Lock-Verbindungsabschnitt (10).

4. Vorrichtung (100, 200, 200`, 300) nach wenigstens einem der vorgenannten Ansprüche, **dadurch gekennzeichnet, dass** eine der beiden Aufnahmeeinrichtungen (20, 320; 30, 130, 330), insbesondere die erste Aufnahmeeinrichtung (20, 320), ortsfest angeordnet ist und die andere Aufnahmeeinrichtung (20, 320; 30, 130, 330), insbesondere die zweite Aufnahmeeinrichtung (30, 130, 330), in axialer Richtung entlang der Verschraubungsachse (V) bewegbar und um die Verschraubungsachse (V) drehbar ist.

5. Vorrichtung (100) nach wenigstens einem der vorgenannten Ansprüche, **dadurch gekennzeichnet, dass** die Vorrichtung (100) einen Spindelantrieb mit einer mittels eines Antriebsmotors antreibbaren Gewindespindel (30) zur Erzeugung der Relativbewegung zwischen der ersten Aufnahmeeinrichtung (20) und der zweiten Aufnahmeeinrichtung (30) aufweist, wobei die Gewindespindel (30) vorzugsweise mittels wenigstens eines ortsfest angeordneten Lagers (30A, 30B) geführt ist.

6. Vorrichtung (100) nach Anspruch 5, **dadurch gekennzeichnet, dass** die erste Aufnahmeeinrichtung (20, 320) oder die zweite Aufnahmeeinrichtung (30), insbesondere die zweite Aufnahmeeinrichtung (30), mit der Gewindespindel (30) drehverbunden oder drehverbindbar ist, wobei die Aufnahmeeinrichtung (30) vorzugsweise in einer radialen Ausnehmung (40) der Gewindespindel (30) angeordnet ist oder durch eine radiale Ausnehmung (40) in der Gewindespindel (30) gebildet ist.

7. Vorrichtung (200, 200') nach wenigstens einem der Ansprüche 1 bis 4, **dadurch gekennzeichnet, dass** die Vorrichtung (200, 200') zur Erzeugung der Relativbewegung zwischen der ersten Aufnahmeeinrichtung (20) und der zweiten Aufnahmeeinrichtung einen ersten Antriebsmotor und ein mittels des ersten Antriebsmotors antreibbares Getriebe mit einem ersten Getriebeelement (130) und wenigstens einem zweiten, mit dem ersten Getriebeelement (130) drehverbundenen Getriebeelement (31) aufweist, wobei die erste Aufnahmeeinrichtung (20) oder die zweite Aufnahmeeinrichtung, insbesondere die zweite Aufnahmeeinrichtung (130), mit dem ersten Getriebeelement (130) drehverbunden oder drehverbindbar ist.

8. Vorrichtung (100, 200, 200`, 300) nach Anspruch 7, **dadurch gekennzeichnet, dass** die Vorrichtung dazu eingerichtet ist, die erste Aufnahmeeinrichtung (20, 320) und die zweite Aufnahmeeinrichtung (30, 130, 330) entlang der Verschraubungsachse (V) zusammenzuschieben, insbesondere zusammenzudrücken, und/oder auseinanderzuziehen.

9. Vorrichtung (300) nach wenigstens einem der Ansprüche 1 bis 4, **dadurch gekennzeichnet, dass** die erste Aufnahmeeinrichtung (320) und/oder die zweite Aufnahmeeinrichtung (330), insbesondere die zweite Aufnahmeeinrichtung, an einem Greiferarm (301, 302) befestigt ist.

10. Vorrichtung (100, 200, 200`, 300) nach wenigstens einem der vorgenannten Ansprüche, **dadurch gekennzeichnet, dass** die Vorrichtung derart ausgebildet ist, dass das erste Verbindungselement (1) und das zweite Verbindungselement (6, 6') der Gewindeverbindung nach dem Herstellen der Gewindeverbindung jeweils in radialer Richtung (R) aus der ersten Aufnahmeeinrichtung (20, 320) und der zweiten Aufnahmeeinrichtung (30, 130, 330) entnehmbar sind.

11. Verfahren zum Herstellen und/oder Lösen einer mit einem Fluid durchströmbaren, medizinischen Gewindeverbindung aus einem ersten Verbindungselement (1) und einem zweiten Verbindungselement (6, 6'), vorzugsweise zum Herstellen und/oder Lösen einer Luer-Lock-Verbindung, mithilfe einer Vorrichtung (100, 200, 200`, 300) mit einer ersten Aufnahmeeinrichtung (20, 320) und einer zweiten Aufnahmeeinrichtung (30, 130, 330), wobei die erste Aufnahmeeinrichtung (20, 320) und die zweite Aufnahmeeinrichtung (30, 130, 330) um eine gemeinsame Verschraubungsachse (V) relativ zueinander drehbar sind und entlang der Verschraubungsachse (V) relativ zueinander verlagerbar sind, insbesondere mithilfe einer gemäß den Ansprüchen 1 bis 10 ausgebildeten Vorrichtung (100, 200, 200`, 300), mit den Schritten:
- Bereitstellen eines ersten Verbindungselementes (1) und eines mit dem ersten Verbindungselement (1) zu verbindenden, zweiten Verbindungselementes (6, 6') oder der zu lösenden Gewindeverbindung,
- Einbringen des ersten Verbindungselementes (1) in die erste Aufnahmeeinrichtung (20, 320) der Vorrichtung (100, 200, 200', 300),
- Einbringen des zweiten Verbindungselementes (6, 6') in die zweite Aufnahmeeinrichtung (30, 130, 330) der Vorrichtung (100, 200, 200', 300),
- Bewegen der ersten Aufnahmeeinrichtung (20, 320) derart relativ zur zweiten Aufnahmeeinrichtung (30, 130, 330), dass das erste Verbindungselement (1) und das zweite Verbindungselement (6, 6') miteinander verschraubt werden oder die Gewindeverbindung gelöst wird,
**dadurch gekennzeichnet, dass** das erste Verbindungselement (1) und/oder das zweite Verbindungselement (6, 6') oder die Gewindeverbindung bezogen auf die Verschraubungsachse (V) in radialer Richtung (R) in die zugehörige Aufnahmeeinrichtung (20, 320; 30, 130, 330) eingebracht wird.

12. Verfahren nach Anspruch 11, **dadurch gekennzeichnet, dass** nach dem Herstellen der Gewindeverbindung das erste Verbindungselement (1) und das zweite Verbindungselement (6, 6') der Gewindeverbindung jeweils in radialer Richtung (R) aus der ersten Aufnahmeeinrichtung (20, 320) und der zweiten Aufnahmeeinrichtung (30, 130, 330) entnommen werden.

13. Kanülierautomat, der eine Vorrichtung gemäß einem der Ansprüche 1 bis 10 aufweist und der insbesondere zur Durchführung eines Verfahrens gemäß einem der Ansprüche 11 bis 12 eingerichtet ist.

## Claims

1. An apparatus (100, 200, 200', 300) for producing and/or disengaging a fluid flow-capable medical threaded connection of a first connecting element (1) and a second connecting element (6, 6`), in particular for producing and/or disengaging a Luer lock connection,
wherein the apparatus (100, 200, 200', 300) comprises a first receiving device (20, 320) for receiving the first connecting element (1) and a second receiving device (30, 130, 330) for receiving the second connecting element (6, 6`),
wherein the first receiving device (20, 320) and the second receiving device (30, 130, 330) are rotatable relative to each other about a common screwing axis (V) and displaceable relative to each other along said screwing axis (V),
and wherein the apparatus (100, 200, 200', 300) is designed to relatively move the first receiving device (20, 320) and the second receiving device (30, 130, 330) toward each other after the introduction of the first connecting element (1) into the first receiving device (20, 320) and the introduction of the second connecting element (6, 6`) into the second receiving device (30, 130, 330) such that the first connecting element (1) and the second connecting element (6, 6') are screwed together or the threaded connection disengaged,
**characterized in that**
the first receiving device (20) and/or the second receiving device (30, 13) has a recess (40) which is designed to receive the associated connecting element (1; 6, 6') and which is outwardly open in the radial direction relative to the screwing axis (V), or
the first receiving device (320) and/or the second receiving device (330) comprises a gripper (303, 304) or is designed as a gripper (303, 304),
so that the associated connecting element (1; 6, 6') can be introduced into the first receiving device (20, 320) and/or the second receiving device (30, 130, 330) in the radial direction (R) relative to the screwing axis (V).

2. The apparatus (100, 200, 200', 300) according to claim 1, **characterized in that** the apparatus (100, 200, 200', 300) comprises an actuator device for generating the relative movement between the first receiving device (20, 320) and the second receiving device (30, 130, 330) for at least semi-automatically produce and/or disengage the threaded connection.

3. The apparatus (100, 200, 200', 300) according to at least one of the preceding claims, **characterized in that** one of the two receiving devices (20, 320; 30, 130, 330), in particular the first receiving device (20, 320), is designed for receiving a first connecting element (1) comprising a first connecting section (3) having a sleeve-shaped internal thread (5) arranged concentric to a first axis (A1), in particular for receiving a first connecting element (1) with a male Luer lock connecting section (3), and the other receiving device (20, 320; 30, 130, 330), in particular the second receiving device (30, 130, 330), is designed for receiving a second connecting element (6, 6') comprising a second connecting section (10) having an external thread (7) arranged concentric to a second axis (A2), in particular for receiving a second connecting element (6, 6') with a female Luer lock connecting section (10).

4. The apparatus (100, 200, 200', 300) according to at least one of the preceding claims, **characterized in that** one of the two receiving devices (20, 320; 30, 130, 330), in particular the first receiving device (20, 320), is arranged in position-fixed manner and the other receiving device (20, 320; 30, 130, 330), in particular the second receiving device (30, 130, 330), is axially displaceable along the screwing axis (V) and rotatable about said screwing axis (V).

5. The apparatus (100) according to at least one of the preceding claims, **characterized in that** the apparatus (100) comprises a spindle drive having a threaded spindle (30) which can be driven by a drive motor for generating the relative movement between the first receiving device (20) and the second receiving device (30), wherein the threaded spindle (30) is preferably guided by means of at least one fixedly disposed bearing (30A, 30B).

6. The apparatus (100) according to claim 5, **characterized in that** the first receiving device (20, 320) or the second receiving device (30), in particular the second receiving device (30), is rotationally connected or connectable to the threaded spindle (30), wherein the receiving device (30) is preferably arranged in a radial recess (40) of the threaded spindle (30) or formed by a radial recess (40) in the threaded spindle (30).

7. The apparatus (200, 200') according to at least one of claims 1 to 4, **characterized in that** the apparatus (200, 200') comprises a first drive motor and a gear mechanism having a first gear element (130) and at least one second gear element (31) rotationally connected to the first gear element (130) which can be driven by means of the first drive motor for generating the relative movement between the first receiving device (20) and the second receiving device, wherein the first receiving device (20) or the second receiving device, in particular the second receiving device (130), is rotationally connected or connectable to the first gear element (130).

8. The apparatus (100, 200, 200', 300) according to claim 7, **characterized in that** the apparatus is designed to push the first receiving device (20, 320) and the second receiving device (30, 130, 330) together along the screwing axis (V), in particular press them together, and/or pull them apart.

9. The apparatus (300) according to at least one of claims 1 to 4, **characterized in that** the first receiving device (320) and/or the second receiving device (330), in particular the second receiving device, is/are mounted on a gripper arm (301, 302).

10. The apparatus (100, 200, 200', 300) according to at least one of the preceding claims, **characterized in that** the apparatus is designed such that the first connecting element (1) and the second connecting element (6, 6`) of the threaded connection can be removed from the first receiving device (20, 320) and the second receiving device (30, 130, 330) in the radial direction (R) after the threaded connection having been made.

11. A method for producing and/or disengaging a fluid flow-capable medical threaded connection of a first connecting element (1) and a second connecting element (6, 6'), preferably for producing and/or disengaging a Luer lock connection, by means of an apparatus (100, 200, 200', 300) comprising a first receiving device (20, 320) and a second receiving device (30, 130, 330), wherein the first receiving device (20, 320) and the second receiving device (30, 130, 330) are rotatable relative to each other about a common screwing axis (V) and displaceable relative to each other along said screwing axis (V), in particular by means of an apparatus (100, 200, 200', 300) designed in accordance with claims 1 to 10, comprising the steps:
- Providing a first connecting element (1) and a second connecting element (6, 6') to be connected to the first connecting element (1) or the threaded connection to be disengaged,
- Introducing the first connecting element (1) into the first receiving device (20, 320) of the apparatus (100, 200, 200', 300),
- Introducing the second connecting element (6, 6') into the second receiving device (30, 130, 330) of the apparatus (100, 200, 200', 300),
- Moving the first receiving device (20, 320) relative to the second receiving device (30, 130, 330) such that the first connecting element (1) and the second connecting element (6, 6') screw together or the threaded connection disengages,
**characterized in that** the first connecting element (1) and/or the second connecting element (6, 6') or the threaded connection is/are introduced into the associated receiving device (20, 320; 30, 130, 330) in the radial direction (R) in relation to the screwing axis (V).

12. The method according to claim 11, **characterized in that** after the threaded connection has been made, the first connecting element (1) and the second connecting element (6, 6') of the threaded connection are in each case removed from the first receiving device (20, 320) and the second receiving device (30, 130, 330) in the radial direction (R).

13. A cannulation robot comprising an apparatus in accordance with one of claims 1 to 10 and which is in particular designed to implement a method according to one of claims 11 to 12.

## Revendications

1. Dispositif (100, 200, 200', 300) pour la création et/ou le desserrage d'un raccord fileté médical à travers lequel peut s'écouler un fluide, composé d'un premier élément de raccord (1) et d'un second élément de raccord (6, 6'), en particulier pour la création et/ou le desserrage d'un raccord Luer-Lock,
dans lequel le dispositif (100, 200, 200', 300) présente un premier équipement de réception (20, 320) pour la réception du premier élément de raccord (1) et un second équipement de réception (30, 130, 330) pour la réception du second élément de raccord (6, 6'),
dans lequel le premier équipement de réception (20, 320) et le second équipement de réception (30, 130, 330) peuvent être tournés l'un par rapport à l'autre autour d'un axe de vissage (V) commun et peuvent être déplacés l'un par rapport à l'autre le long de l'axe de vissage (V),
et dans lequel le dispositif (100, 200, 200', 300) est configuré pour déplacer, après l'introduction du premier élément de raccord (1) dans le premier équipement de réception (20, 320) et l'introduction du second élément de raccord (6, 6') dans le second équipement de réception (30, 130, 330), le premier équipement de réception (20, 320) et le second équipement de réception (30, 130, 330) l'un par rapport à l'autre de sorte que le premier élément de raccord (1) et le second élément de raccord (6, 6') soient vissés ensemble ou que le raccord fileté soit desserré,
**caractérisé en ce que**
le premier équipement de réception (20) et/ou le second équipement de réception (30, 13) présente un évidement (40) ouvert vers l'extérieur dans la direction radiale par rapport à l'axe de vissage (V) et conçu pour la réception de l'élément de raccord (1 ; 6, 6') correspondant, ou
le premier équipement de réception (320) et/ou le second équipement de réception (330) présente un préhenseur (303, 304) ou est conçu en tant que préhenseur (303, 304),
de sorte que l'élément de raccord (1 ; 6, 6') correspondant puisse être introduit dans le premier équipement de réception (20, 320) et/ou dans le second équipement de réception (30, 130, 330) dans la direction radiale (R) par rapport à l'axe de vissage (V).

2. Dispositif (100, 200, 200', 300) selon la revendication 1, **caractérisé en ce que** le dispositif (100, 200, 200', 300) présente un équipement-actionneur en vue de la production du mouvement relatif entre le premier équipement de réception (20, 320) et le second équipement de réception (30, 130, 330) pour la création et/ou le desserrage au moins partiellement automatisés du raccord fileté.

3. Dispositif (100, 200, 200', 300) selon au moins l'une des revendications précédentes, **caractérisé en ce que** l'un des deux équipements de réception (20, 320 ; 30, 130, 330), en particulier le premier équipement de réception (20, 320), est configuré pour la réception d'un premier élément de raccord (1) avec une première section de raccord (3) avec un filetage intérieur (5) en forme de douille et agencé de façon concentrique par rapport à un premier axe (A1), en particulier pour la réception d'un premier élément de raccord (1) avec une section de raccord (3) Luer-Lock mâle, et l'autre équipement de réception (20, 320 ; 30, 130, 330), en particulier le second équipement de réception (30, 130, 330), pour la réception d'un second élément de raccord (6, 6') avec une seconde section de raccord (10) avec un filetage extérieur (7) agencé de façon concentrique par rapport à un second axe (A2), en particulier pour la réception d'un second élément de raccord (6, 6') avec une section de raccord (10) Luer-Lock femelle.

4. Dispositif (100, 200, 200', 300) selon au moins l'une des revendications précédentes, **caractérisé en ce que** l'un des deux équipements de réception (20, 320 ; 30, 130, 330), en particulier le premier équipement de réception (20, 320), est agencé de façon stationnaire et l'autre équipement de réception (20, 320 ; 30, 130, 330), en particulier le second équipement de réception (30, 130, 330), peut être déplacé dans la direction axiale le long de l'axe de vissage (V) et peut être tourné autour de l'axe de vissage (V).

5. Dispositif (100) selon au moins l'une des revendications précédentes, **caractérisé en ce que** le dispositif (100) présente un entraînement à broche avec une broche filetée (30) pouvant être entraînée au moyen d'un moteur d'entraînement pour la production du mouvement relatif entre le premier équipement de réception (20) et le second équipement de réception (30), dans lequel la broche filetée (30) est de préférence guidée au moyen d'au moins un palier (30A, 30B) agencé de façon stationnaire.

6. Dispositif (100) selon la revendication 5, **caractérisé en ce que** le premier équipement de réception (20, 320) ou le second équipement de réception (30), en particulier le second équipement de réception (30), est relié en rotation ou peut être relié en rotation avec la broche filetée (30), dans lequel le second équipement de réception (30) est de préférence agencé dans un évidement (40) radial de la broche filetée (30) ou formé par un évidement (40) radial dans la broche filetée (30).

7. Dispositif (200, 200') selon au moins l'une des revendications 1 à 4, **caractérisé en ce que** le dispositif (200, 200') présente, pour la production du mouvement relatif entre le premier équipement de réception (20) et le second équipement de réception, un premier moteur d'entraînement et une transmission pouvant être entraînée au moyen du premier moteur d'entraînement avec un premier élément de transmission (130) et au moins un deuxième élément de transmission (131) relié en rotation avec le premier élément de transmission (130), dans lequel le premier équipement de réception (20) ou le second équipement de réception, en particulier le second équipement de réception (130), est relié en rotation ou peut être relié en rotation avec le premier élément de transmission (130).

8. Dispositif (100, 200, 200', 300) selon la revendication 7, **caractérisé en ce que** le dispositif est configuré pour pousser ensemble, en particulier comprimer ensemble et/ou tirer pour les séparer, le premier équipement de réception (20, 320) et le second équipement de réception (30, 130, 330) le long de l'axe de vissage (V).

9. Dispositif (300) selon au moins l'une des revendications 1 à 4, **caractérisé en ce que** le premier équipement de réception (320) et/ou le second équipement de réception (330), en particulier le second équipement de réception, est fixé contre un bras de préhenseur (301, 302).

10. Dispositif (100, 200, 200', 300) selon au moins l'une des revendications précédentes, **caractérisé en ce que** le dispositif est conçu de sorte que le premier élément de raccord (1) et le second élément de raccord (6, 6') du raccord fileté peuvent être prélevés après la création du raccord fileté dans la direction radiale (R) respectivement depuis le premier équipement de réception (20, 320) et le second équipement de réception (30, 130, 330).

11. Procédé de création et/ou de desserrage d'un raccord fileté médical à travers lequel peut s'écouler un fluide, composé d'un premier élément de raccord (1) et d'un second élément de raccord (6, 6'), de préférence pour la création et/ou le desserrage d'un raccord Luer-Lock, à l'aide d'un dispositif (100, 200, 200', 300) avec un premier équipement de réception (20, 320) et un second équipement de réception (30, 130, 330), dans lequel le premier équipement de réception (20, 320) et le second équipement de réception (30, 130, 330) peuvent être tournés l'un par rapport à l'autre autour d'un axe de vissage (V) commun et peuvent être déplacés l'un par rapport à l'autre le long de l'axe de vissage (V), en particulier à l'aide d'un dispositif (100, 200, 200', 300) conçu selon les revendications 1 à 10, avec les étapes de :
- fourniture d'un premier élément de raccord (1) et d'un second élément de raccord (6, 6') à relier avec le premier élément de raccord (1) ou du raccord fileté à desserrer,
- introduction du premier élément de raccord (1) dans le premier équipement de réception (20, 320) du dispositif (100, 200, 200', 300),
- introduction du second élément de raccord (6, 6') dans le second équipement de réception (30, 130, 330) du dispositif (100, 200, 200', 300),
- déplacement du premier équipement de réception (20, 320) par rapport au second équipement de réception (30, 130, 330) de sorte que le premier élément de raccord (1) et le second élément de raccord (6, 6') soient vissés ensemble ou que le raccord fileté soit desserré,
**caractérisé en ce que** le premier élément de raccord (1) et/ou le second élément de raccord (6, 6') ou le raccord fileté est introduit dans l'équipement de réception (20, 320 ; 30, 130, 330) correspondant dans la direction radiale (R) par rapport à l'axe de vissage (V).

12. Procédé selon la revendication 11, **caractérisé en ce que** le premier élément de raccord (1) et le second élément de raccord (6, 6') du raccord fileté sont prélevés après la création du raccord fileté dans la direction radiale (R) respectivement depuis le premier équipement de réception (20, 320) et le second équipement de réception (30, 130, 330).

13. Automate de canulation qui présente un dispositif selon l'une des revendications 1 à 10 et qui est en particulier configuré pour exécuter un procédé selon l'une des revendications 11 à 12.
